# EUROPEAN PATENT SPECIFICATION

(11) **EP 1 516 184 B1**
(45) Date of publication and mention of the grant of the patent: **01.08.2007**
(21) Application number: 03760107.7
(22) Date of filing: 18.06.2003
(51) Int. Cl.: G01N 33/50, A61K 38/10, A61K 38/17, C12N 15/62, C12N 15/85, C07K 7/08, C07K 14/47

(54) **Cell-selective delivery system**
Zell-selektives Abgabe System
Système de délivrance vers une cellule spécifique

(30) Priority: 18.06.2002 SE 0201863; 25.06.2002 US 391788 P
(43) Date of publication of application: 23.03.2005
(73) Proprietor: CePep II AB, 104 30 Stockholm (SE)
(72) Inventor: HÄLLBRINK, Mattias, S-113 28 Stockholm (SE); POOGA, Margus, E-51010 Tartu (EE); METSIS, Madis, Tallin University of Technology, 12618 Tallinn (EE); KOGERMAN, Priit, E-79601 Tabasalu (EE); VALKNA, Andreas, Viimsi (EE); MEIKAS, Anne, Saku Estonia (EE); LINDGREN, Maria, S-113 28 Stockholm (SE); GRÄSLUND, Astrid, S-191 44 Sollentuna (SE); ERIKSSON, Göran, S-116 43 Stockholm (SE); ÖSTENSSON, Claes, Göran, S-171 64 Solna (SE); BUDIHNA, Metka, 1210 Ljubljana-Sentvid (SI); ZORKO, Matjaz, S-1291 Skofljica (SI); ELMQUIST, Anna, S-761 71 Norrtälje (SE); SOOMETS, Ursel, Tartu (EE); LUNDBERG, Pontus, S-169 72 Solna (SE); JÄRVER, Peter, S-116 67 Stockholm (SE); SAAR, Külliki, S-113 53 Stockholm (SE); EL-ANDALOUSSI, Samir, S-104 05 Stockholm (SE); KILK, Kalle, S-182 38 Danderyd (SE); LANGEL, Ülo, S-124 32 Bandhagen (SE)
(74) Representative: Nilsson, Brita Linnea
(86) International application number: PCT/IB2003/003163
(87) International publication number: WO 2003/106491

(56) References cited:
- WO-A-00/34308
- WO-A-02/02595
- WO-A-02/18572
- WO-A-02/053583
- WO-A-02/062823
- WO-A-03/064453
- US-A1- 2002 031 818
- SANDBERG M ET AL: "NEW CHEMICAL DESCRIPTORS RELEVANT FOR THE DESIGN OF BIOLOGICALLY ACTIVE PEPTIDES. A MULTIVARIATE CHARACTERIZATION OF 87 AMINO ACIDS" JOURNAL OF MEDICINAL CHEMISTRY, AMERICAN CHEMICAL SOCIETY. WASHINGTON, US, vol. 41, 1998, pages 2481-2491, XP000877493 ISSN: 0022-2623 cited in the application
- LINDGREN M M ET AL: "Cell-penetrating peptides" TRENDS IN PHARMACOLOGICAL SCIENCES, ELSEVIER TRENDS JOURNAL, CAMBRIDGE, GB, vol. 21, no. 3, March 2000 (2000-03), pages 99-103, XP004202572 ISSN: 0165-6147 cited in the application
- DEROSSI ET AL: "Trojan peptides: the penetratin system for intracellular delivery" TRENDS IN CELL BIOLOGY, ELSEVIER SCIENCE LTD, XX, vol. 8, no. 2, February 1998 (1998-02), pages 84-87, XP002122131 ISSN: 0962-8924 cited in the application
- VIVES E ET AL: "A TRUNCATED HIV-1 TAT PROTEIN BASIC DOMAIN RAPIDLY TRANSLOCATES THROUGH THE PLASMA MEMBRANE AND ACCUMULATES IN THE CELL NUCLEUS" JOURNAL OF BIOLOGICAL CHEMISTRY, AMERICAN SOCIETY OF BIOLOGICAL CHEMISTS, BALTIMORE, MD, US, vol. 272, no. 25, 20 June 1997 (1997-06-20), pages 16010-16017, XP002940007 ISSN: 0021-9258
- MI Z ET AL: "CHARACTERIZATION OF A CLASS OF CATIONIC PEPTIDES ABLE TO FACILITATEEFFICIENT PROTEIN TRANSDUCTION IN VITRO AND IN VIVO" MOLECULAR THERAPY, ACADEMIC PRESS, SAN DIEGO, CA,, US, vol. 2, no. 4, October 2000 (2000-10), pages 339-347, XP001004549 ISSN: 1525-0016
- DOKKA SUJATHA ET AL: "Cellular delivery of oligonucleotides by synthetic import peptide carrier" PHARMACEUTICAL RESEARCH (NEW YORK), vol. 14, no. 12, December 1997 (1997-12), pages 1759-1764, XP008035822 ISSN: 0724-8741
- KIRCHEIS R ET AL: "Design and gene delivery activity of modified polyethylenimines." ADVANCED DRUG DELIVERY REVIEWS. 31 DEC 2001, vol. 53, no. 3, 31 December 2001 (2001-12-31), pages 341-358, XP001064460 ISSN: 0169-409X
- HASHIDA H ET AL: "Fusion of HIV-1 Tat protein transduction domain to poly-lysine as a new DNA delivery tool" BRITISH JOURNAL OF CANCER, vol. 90, no. 6, 22 March 2004 (2004-03-22), pages 1252-1258, XP002297597 ISSN: 0007-0920
- TR¹HIN RACHEL ET AL: "Chances and pitfalls of cell penetrating peptides for cellular drug delivery." EUROPEAN JOURNAL OF PHARMACEUTICS AND BIOPHARMACEUTICS : OFFICIAL JOURNAL OF ARBEITSGEMEINSCHAFT FUR PHARMAZEUTISCHE VERFAHRENSTECHNIK E.V. SEP 2004, vol. 58, no. 2, September 2004 (2004-09), pages 209-223, XP002297598 ISSN: 0939-6411
- IGNATOVICH IRINA A ET AL: "Complexes of plasmid DNA with basic domain 47-57 of the HIV-1 Tat protein are transferred to mammalian cells by endocytosis-mediated pathways." JOURNAL OF BIOLOGICAL CHEMISTRY, vol. 278, no. 43, 24 October 2003 (2003-10-24), pages 42625-42636, XP002297599 ISSN: 0021-9258
- PERALES J C ET AL: "An evaluation of receptor-mediated gene transfer using synthetic DNA-ligand complexes" EUROPEAN JOURNAL OF BIOCHEMISTRY, BERLIN, DE, vol. 226, no. 2, December 1994 (1994-12), pages 255-266, XP002099634 ISSN: 0014-2956
- WAGNER ET AL: "Transferrin-polycation-DNA complexes: the effect of polycations on the structure of the complex and DNA delivery to cells" PROCEEDINGS OF THE NATIONAL ACADEMY OF SCIENCES OF USA, NATIONAL ACADEMY OF SCIENCE. WASHINGTON, US, vol. 88, no. 10, 1991, pages 4255-4259, XP002001450 ISSN: 0027-8424
- SINGH ET AL: "PEPTIDE-BASED INTRACELLULAR SHUTTLE ABLE TO FACILITATE GENE TRANSFER IN MAMMALIAN CELLS" BIOCONJUGATE CHEMISTRY, AMERICAN CHEMICAL SOCIETY, WASHINGTON, US, vol. 10, no. 5, September 1999 (1999-09), pages 745-754, XP000854014 ISSN: 1043-1802

## Description

### FIELD OF THE INVENTION

The present invention relates to a cell-selective delivery system for a cargo, such as a cytostatic and/or cytotoxic agent, to be delivered to a target cell. The invention also relates to the cell-selective delivery system for use in the manufacture of a medicament.

### BACKGROUND OF THE INVENTION

A number of techniques have been developed to deliver different cellular effectors into cells. The majority of these techniques are invasive, like electroporation or microinjection. Liposome encapsulation and receptor-mediated endocytosis are milder methods, but they unfortunately suffer from serious drawbacks, in particular, low delivery yield.

The established view in cellular biology dictates that the cellular internalisation of hydrophilic macromolecules can only be achieved through the classical endocytosis pathway. However, in the last decade, several peptides have been demonstrated to translocate across the plasma membrane of eukaryotic cells by a seemingly energy-independent pathway. These peptides are defined as cell-penetrating peptides (CPPs) and have been used successfully for intracellular delivery of macromolecules with molecular weights several times greater than their own. (M. Lindgren et al, 2000, Cell-penetrating peptides, TIPS, Vol. 21, pg. 99-103)

Cellular delivery using these cell-penetrating peptides offers several advantages over conventional techniques. It is non-invasive, energy-independent, is efficient for a broad range of cell types and can be applied to cells *en masse*. Furthermore, it has been found that for certain types of CPPs, cellular internalisation occurs at 37°C, as well as at 4°C and that it can not be saturated. Also, in most cases, the internalisation seems not to require a chiral receptor protein, since no enantiomeric discrimination has been observed.

Until recently, transport of hydrophilic macromolecules into the cytoplasmic and nuclear compartments of living cells without disrupting the plasma membrane seemed a far-off goal. Because of their low biomembrane permeability and their relatively rapid degradation, polypeptides and oligonucleotides were generally considered to be of limited therapeutic value. This is an obstacle in both biomedical research and the pharmaceutical industry.

An even more difficult, although very important task, is to deliver hydrophilic macromolecules across the blood-brain barrier. Several methods have been envisaged to overcome this hurdle. Nevertheless, they all suffer from limitations, such as their effectiveness being restricted to a subset of molecules, or that they give a too low yield. However, recent reports suggest that CPPs might be able to transport macromolecules across the blood-brain barrier.

Another essential area for desired delivery of effectors is nuclear import, wherein, in general, it has been found that the signal sequence must contain some positively charged (basic) residues (Moroianu J., J.Cell Biochem, 1999). It seems that such charged amino acids might also be required for plasma membrane translocation.

Today, a diversity of cell-penetrating peptides, CPPs, is known. Several peptides have been demonstrated to translocate across the plasma membrane of eukaryotic cells by a seemingly energy-independent pathway. Thus, cell-penetrating peptides might be used as delivery vectors for pharmacologically interesting substances, such as peptides, proteins, oligonucleotides, antisense molecules, as well as for research tools.

Of particular interest among CPPs are those peptides that have low lytic activity. These translocating peptides, also known as Trojan peptides (D. Derossi et al., Trends Cell Biol. 8 (1998) 84-87), have been applied as vectors for the delivery of hydrophilic biomolecules and drugs into cytoplasmic and nuclear compartments of cells, both *in vivo* and *in vitro* (for review, see M. Lindgren et al., Trends Pharmacol. Sci. 21 (2000) 99-103). When covalently linked with a cargo, including polypeptides and oligonucleotides with many times their own molecular mass, these peptides are still able to translocate.

Examples of useful transport peptides are sequences derived from homeodomains of certain transcription factors, as well as so-called Tat-derived peptides and peptides based on signal sequences. The first of the homeodomain-derived translocating peptides was penetratin (SEQ ID NO: 1), denoted pAntp, with a sequence corresponding to the 16 residues of the third α-helix (residues 43-58) from the Antennapedia homeodomain protein of Drosophila (D. Derossi et al., J. Biol. Chem. 269 (1994) 10444-10450; A. Prochiantz, Ann. NY Acad. Sci. 886 (1999) 172-179). The pAntp peptide retains its membrane translocation properties and has therefore been proposed to be a universal intercellular delivery vector (D. Derossi et al., Trends Cell Biol. 8 (1998) 84-87).

Purely synthetic or chimeric peptides have also been designed, as reviewed in (D. Derossi et al., Trends Cell Biol. 8 (1998) 84-87, and M. Lindgren et al., Trends Pharmacol. Sci. 21 (2000) 99-103).

Transportan (SEQ ID NO: 2), e.g., a non-natural peptide, is able to deliver an antibody molecule with a molecular mass of about 150 kDa over the plasma membrane, although Transportan itself is only a 3 kDa peptide. Transportan and penetratin were demonstrated to deliver a non-natural DNA analogue, PNA (peptide nucleic acid) into cytoplasm and nuclei of cells in culture (Pooga et al. 1998, Nature Biotech.).

Another group of peptides that have surprisingly been shown to be able to transport across the cellular membrane, when coupled to a hydrophobic moiety, are modified receptors, in particular G protein coupled receptors, which are called pepducines (see e.g. WO0181408, Kuliopulos, et.al.). It was discovered that attachment of a hydrophobic moiety to peptides derived from the third intracellular loop of a 7TM receptor yields cellular translocation of said chimeric peptides and full agonist and/or antagonist of receptor G-protein signalling. These pepducines are membrane inserting, membrane-tethered chimeric peptides and require the presence of their cognate receptor for activity and are highly selective for receptor type.

Although their astonishing transport capability has put CPPs into the focus of scientific interest for the last years, the most basic mechanisms of translocation for the different CPPs is still unknown. For instance, it is today still not known in the field, whether any particular secondary structure has to be induced in order to allow (energetically) a translocation, involving a concomitant transient membrane destabilization. It is clear, however, that the molecular details of the peptide-membrane interactions must be of fundamental importance for the translocation process.

The mechanism and requirements for internalisation have been studied on interactions between amphipathic α-helical peptides and lipid (bi)layers. The results of these studies often suggest tryptophan to be responsible for internalisation of a peptide, but although aromatic amino acids may be preferred in CPP sequences, they are not absolutely necessary for cell penetration.

Apart from the cell penetration capability, little correlation of structure or behaviour has been found between CPPs. Up to now, CPPs have thus not been designed in a rational manner, but have been found serendipitously. However, the sequences of CPPs published so far have a positive net-charge as the only common feature, giving a starting point for the prediction of CPP functionality in a given peptide sequence. Clearly, though, all sequences with a positive net-charge cannot be cell-penetrating, indicating that further restrictions are needed to select CPPs with any certainty.

WO0034308 describes a protein transduction system comprising one or more fusion proteins that includes a transduction domain and a cytotoxic domain. The protein transduction system effectively kills or injures cells infected by one or a combination of different pathogens or cells exhibiting unique characteristics such as high levels of heavy metals, DNA damage or uncontrolled cell division.

### DESCRIPTION OF THE INVENTION

The present invention provides a cell-selective delivery system as defined in the appended claim 1 and subclaims as well as the cell-selective delivery system for use in the manufacture of a medicament as defined in claim 8.

In the present context, an amino acid is any organic compound containing an amino (-NH₂) and a carboxyl (-COOH) group. Amino acids can be in either L- or D- form. There are at present 22 known coded α-amino acids from which proteins are synthesized during ribosomal translation of mRNA. Additionally, a vast number of non-coded amino acids are constantly emerging. Both coded and non-coded amino acids can of course be part of the amino acid sequences, peptide fragments, peptides, proteins and/or polypeptides included in the present invention.

Amino acid sequence is in the present context the precisely defined linear order of amino acids (including both coded and/or non-coded amino acids) in a peptide fragment, peptide, protein or polypeptide.

The term non-peptide analogue is in the present context employed to describe any amino acid sequence comprising at least one non-coded amino acid and/or having a backbone modification resulting in an amino acid sequence without a peptide linkage, i.e. a CO-NH bond formed between the carboxyl group of one amino acid and the amino group of another amino acid.

Furthermore, the present amino acid sequences may either be amidated or occur as free acids.

Reporter groups of different characters can be coupled to a putative CPP in order to estimate its cellular translocation and efficiency, such as biotin and different fluorophores, e.g. fluorescein, aminobenzoic acid, and rhodamines.

The term "cell-penetrating capacity" of a peptide will henceforth be used synonymously to its capability to translocate across the plasma membrane into either cytoplasmic and/or nuclear compartments of eukaryotic and/or prokaryotic cells, such as into cytoplasm, nucleus, lysosome, endoplasmatic reticulum, golgi apparatus, mitocondria and/or chloroplast, seemingly energy-independently. Additionally, the term "cell-penetrating capacity" of a peptide can in some aspects of the invention also be used synonymously to indicate transcellular or transmembrane transport, and thus also stand for e.g. the capability to translocate across an epithelial membrane, such as across the epithelium in the intestinal/buccal system, the mucosa in the mouth, lung, rectum or nose, or the blood-brain barrier of a mammal.

A detected or *de novo* designed and verified peptide, displaying cellular penetration capacity according to the present invention is in the present context defined as a "cell-penetrating peptide (CPP)" and can e.g. be used for intracellular delivery of macromolecules, such as polypeptides and/or oligonucleotides with molecular weights several times greater than its own.

In general, cellular delivery using a cell-penetrating peptide and/or a non-peptide analogue thereof is non-invasive, energy-independent, efficient for a broad range of cell types and/or a broad variety of cargo, applicable to cells *en masse,* non-saturable, and/or receptor independent.

CPPs detected or *de novo* designed, and/or verified by a method disclosed in the present application will be useful for the transport of hydrophilic macromolecules into the cytoplasmic and nuclear compartments of a living cell and/or microorganism, without permanently disrupting the plasma membrane, as well as for delivering hydrophilic macromolecules across the blood-brain barrier, permitting e.g. the intracellular transport of conjugated oligopeptides and oligonucleotides and drugs.

Thus, a cell-penetrating peptide and/or a non-peptide analogue thereof might in the present context be used as a delivery vector for any pharmacologically interesting substance, such as a peptide, polypeptide, protein, small molecular substance, drug, mononucleotide, oligonucleotide, polynucleotide, antisense molecule, double stranded as well as single stranded DNA, RNA and/or any artificial or partly artificial nucleic acid, e.g. PNA, as well as a research tool for delivering e.g. tags and markers and/or for changing membrane potentials and/or properties.

A CPP found or designed and/or produced according to the present invention can therefore be of use as a vector for the delivery of a hydrophilic biomolecule and/or drug into cytoplasmic and nuclear compartments of a cell and/or a tissue, both *in vivo* and *in vitro.*

When covalently linked with a cargo, including any peptide, polypeptide, protein, small molecular substance, drug, polypeptide and oligonucleotide, with many times its own molecular mass, a CPP might still be able to translocate.

What is more, a CPP can in itself display intra and/or extracellular effector activity, thus function as a cell-penetrating functional protein-mimicking peptide, or even display a new, non-predictable function when designed *de novo.*

A cellular effector can herein be either an intracellular and/or extracellular effector and is in the present context defined as a structure that produces a cellular effect, such as a contraction, secretion, electrical impulse, or activation or inactivation of an intracellular and/or extracellular signalling cascade, or that induces the up regulation of a cellular level of an mRNA and/or a protein, in response to a stimulation by said effector. A typical effector is in the present context selected from the group consisting of a metabolite, an antagonist, an agonist, a receptor ligand, a receptor coupled protein, an activated receptor, an enzyme inhibitor, activator/inactivator and/or stimulator, a kinase, a phosphatase, an enhancer, or a silencer, a transcription factor, a transporter and/or a transmitter, a hormone, a channel, an ion, a prion, and a viral protein.

### Cargo

As described previously, a CPP can be coupled to a cargo to function as a carrier of said cargo into cells, various cellular compartments, tissue or organs. The cargo may be selected from the group consisting of any pharmacologically interesting substance, such as a peptide, polypeptide, protein, small molecular substance, drug, mononucleotide, oligonucleotide, polynucleotide, antisense molecule, double stranded as well as single stranded DNA, RNA and/or any artificial or partly artificial nucleic acid, e.g. PNA, a low molecular weight molecule, saccharid, plasmid, antibiotic substance, cytotoxic and/or antiviral agent. Furthermore, the transport of cargo can be useful as a research tool for delivering e.g. tags and markers as well as for changing membrane potentials and/or properties, the cargo may e.g. be a marker molecule, such as biotin.

With respect to the intended transport of a cargo across the blood-brain barrier, both intracellular and extracellular substances are equally preferred cargo.

In a preferred embodiment of the invention, the cell-penetrating peptide is coupled by a S-S bridge to said cargo. Naturally, there are a broad variety of methods for coupling a cargo to a CPP, selected individually depending on the nature of CPP, cargo and intended use. A mode for coupling can be selected from the group consisting of covalent and non-covalent binding, as biotin-avidin binding, ester linkage, amide bond, antibody bindings, etc.

In some embodiments, a labile binding is preferred, in other embodiments, a stabile binding is elementary, such as in the use of a CPP according to the present invention for use in transport of medical substances, due to the necessary storage of said pharmaceutical compositions before use.

Illustrative examples for the above described embodiments are given in examples 2, 5 and 6.

In example 5, a methotrexate (MTX) conjugate with a CPP carrier is described.
MTX is a cytotoxic drug, which was developed for the treatment of malignancies but is now also used to treat autoimmune diseases, such as psoriasis. Normally, MTX is present in bodily fluids as negatively charged molecule. Therefore, it can cross the cell membrane only with difficulty.

Thus one illustrative example of the use of a CPP for the transport of a cargo according to the present invention comprises MTX-CPP conjugates essentially as comprised in SEQ.ID.NO: 3 - 7, which are e.g. synthesised using a solid phase peptide synthesis strategy as described in example 5, specific examples of which are listed in table 1 below:

**Table 1. MTX-CPP conjugates**

| |
|---|
| Apa-(γGlu)2-Gly-CPP |
| Apa-(γGlu)2-5-Gly-CPP |
| Apa-Cys-S-S-Cys-CPP |

Another example of a molecule being carried across a cellular membrane with a CPP is given in example 6, wherein siRNA uptake is substantially improved.

ln recent years small interfering RNA (siRNA) have gained attention for their highly sensitive ability to regulate gene expression in mammalian cells. siRNA are short strands (about 21-23bp) of double stranded RNA that induce specific cleavage of their complementary mRNA through activation of the RNA-induced silencing complex (RISC). Although RNA-induced silencing is an endogenous mechanism, synthetically synthesized siRNA's could be shown to have the same effect both *in vitro* and *in vivo.*

A well known problem when using siRNA, is low yield of uptake in the cell. By coupling cell-penetrating peptides (CPP) to synthetically synthesized siRNA though, the cellular uptake is significantly improved.

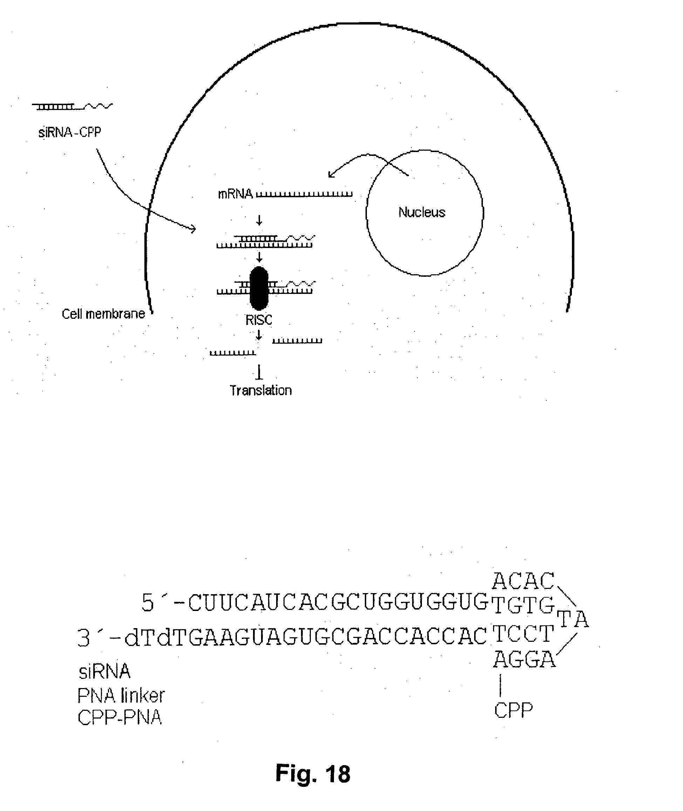

Another specific embodiment of the use of a CPP for cargo transport included in the present invention thus relates to a siRNA against the GALR-1 mRNA coupled to a CPP, such as Transportan10 (Tp10) via a disulfide linker, e.g. as shown in Fig.18 and listed as SEQ ID NO: 2.

### Cell-selective CPPs

In yet a further embodiment of the present invention, a cell-penetrating peptide and/or a non-peptide analogue thereof is provided that will enter selectively into a certain cell type/tissue/organ, or that transports a cargo that will only be activated in a certain cell type, tissue, or organ type.

The inventors show that different CPPs are internalised by specific cell lines, such as human melanoma cell line Bowes and others, with significantly different efficacy and rate of uptake, sometimes more than two-fold. This is a prerequisite to define CPPs that are internalised with different efficacy to different cell lines and tissues. Hence, an important method that may be used in connection with the present invention is the development of selective CPPs (selCPPs) characterised e.g. by testing all available natural or *de novo* designed CPPs for cellular uptake in cell lines, cells, tissues and/or organs into which a selective transport is required. On the other hand, certain selective methods might be employed to artificially enhance the cell selectivity of a CPP of choice for a certain target cell or target cell population, which will be described in detail below.

As an example, cancer cells expose many cell surface antigens and/or proteins, as well as secrete certain proteins. Usually, tumour cells do not express cell surface markers that are unique but rather over-express common receptors/markers. The signalling through these over-expressed markers and/or over-amplifications of the intracellular and/or extracellular signals is thought to be one of the mechanisms for the loss of control of the cellular machinery over the cell cycle. Thus, in a specific embodiment of the present invention, an over-expressed cell surface protein and/or secreted protein is applied as target for CPP addressing.

In one embodiment of the invention, a cell-selective CPP (selCPP) is envisioned that comprises an antigen/protein raised against a cellular marker, selected from the group consisting of channel receptors, tyrosine kinase receptors (e.g. EGF, IGF), guanylate cyclase receptors, serine/threonine kinase receptors, cytokine receptors, receptors coupled to guanosine triphosphate (GTP)-binding proteins (G protein-coupled receptors: GPCRs), glycosphingolipids, CD44, neuropeptide receptors, e.g. neurotensin receptors, galanin, and substance P receptors.

Generally, a cell-selective CPP (selCPP) will of course be extremely useful in the targeted transport of any kind of drug or pharmaceutical substance to a variety of specific eukaryotic and/or prokaryotic cellular targets. A cell-selective transport of such cargo is e.g. envisioned for an improved treatment or prevention of infectious diseases, such as diseases caused by a viral, bacterial or parasital infection.

In order to avoid non-specific internalisation of CPPs before finding a target cell *in vivo,* the invention in a further aspect relates to a new variation of the enzyme-prodrug strategy, wherein a selCPP-conjugate is designed so that the cell penetration-active structure of the CPPs is disrupted until the binding event of a peptide part of said selCPP to a cell/tissue or organ specific receptor/marker, or the cleavage of said selCPP-conjugate by a protease secreted by the target cell/tissue or organ, releases the CPP from conformational discrimination.

The term "enzyme-prodrug strategy/therapy" is in the field of the art used to define a specific approach to delivering a drug, which is focused on the development of amino-acid or nucleic-acid prodrugs, which, before or after delivery, require activation by more or less tissue, organ and/or cell-selective enzymes. Large differences in selectivity are found in the prior art. For some prodrugs, a rapid removal of the released drug from the target tissue/organ/cell explains the low selectivity, whereas for others, cleavage in non-target tissue and insufficient transport across the cell to the enzyme site seems mainly responsible.

Especially many anticancer agents are severely toxic, which explains the need of more effective and less toxic prodrugs and/or softdrugs. A typical prodrug/softdrug must therefore be an efficient and selective substrate for the activating enzyme, and be metabolised to a potent cytotoxin and/or cytostatica, which is preferably able to kill cells at all stages of the cell cycle. Many of the early antimetabolite-based prodrugs provided very polar activated forms that had limited abilities to diffuse across cell membranes, and relied on gap junctions between cells for their bystander effects. Prodrugs as described in the present invention, though, have good distributive properties and their activated species are naturally cell penetrating, so that the resulting bystander effects can maximize the effectiveness of the therapy.

In the present context, the term "enzyme-prodrug strategy/therapy" is additionally used to describe the above revealed method of delivering a drug, wherein the drug itself or its transporter CPP is rendered non-cell-penetrating in order to avoid non-specific internalisation of CPPs before finding a target cell *in vivo,* and wherein only the binding event of a peptide part of said selCPP to a cell/tissue or organ specific receptor/marker, or the cleavage of said selCPP-conjugate by a protease secreted by the target cell/tissue or organ, releases the CPP from conformational discrimination, whereupon it can penetrate the target cell.

An especially preferred embodiment of the present invention thus relates to a cell-selective delivery system for a cytostatic and/or cytotoxic agent, comprising a) a protease consensus site for a protease specifically overexpressed in a target cell, b) a cell-penetrating peptide and/or a non-peptide analogue thereof, and c) a cytostatic and/or cytotoxic agent, wherein said cell-selective delivery system additionally comprises an inactivation sequence repressing the cellular penetration capacity of said cell-penetrating peptide, and which is cleaved by said protease specifically overexpressed in the target cell upon introducing said cell-selective delivery system in the near vicinity of said target cell. See e.g. example 4.

A typical example for the above concept are matrix metallo proteases (MMPs), which are Zn²⁺ metallo endopeptidases. The family contains both membrane bound and secreted members of which both catalyse the breakdown of proteins located either on the cell's plasma membrane or within the extracellular matrix (ECM) (M.D. Sternlicht and Z. Werb " How matrix metallo proteinases regulate cell behavior" Annual Review of Cell and Developmental Biology, 17:463-516, 2001). MMPs have been linked to the invasive and metastatic behaviour of a wide variety of malignancies, and these enzymes are generally overexpressed in a variety of tumours (M.D. Sternlicht and Z. Werb "How matrix metallo proteinases regulate cell behavior" Annual Review of Cell and Developmental Biology, 17:463-516; D.V. Rozanov et al., "Mutation analysis of membrane type-1 metalloproteinase (MT1-MMP, alternative name MMP-14)", Journal of Biological Chemistry (JBC), 276:25705-14, July 13, 2001). Membrane type MMPs (MT-MMP), such as MMP-MT1 have been strongly implicated in oncogenesis. These enzymes localise to the invasive fronts. The soluble MMPs 1-3 and 9 have also been implicated as agonists of tumourigenesis (Smith, L.E., Parks, K.K., Hasegawa, L.S., Eastmond, D.A. & Grosovsky, A.J. Targeted breakage of paracentromeric heterochromatin induces chromosomal instability. Mutagenesis 13, 435-43. (1998)).

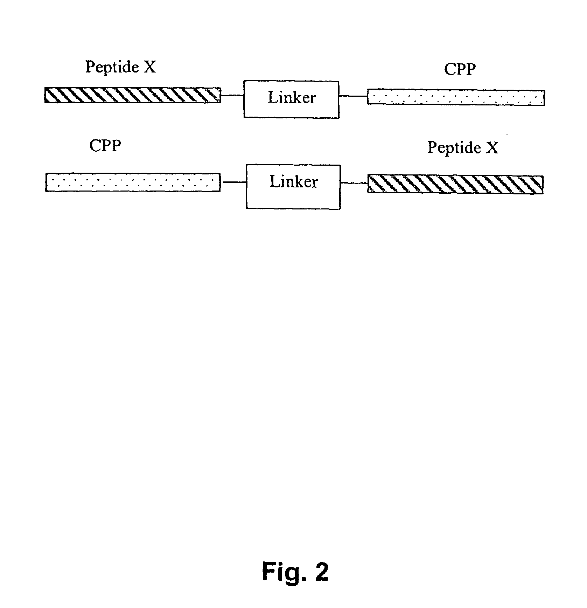

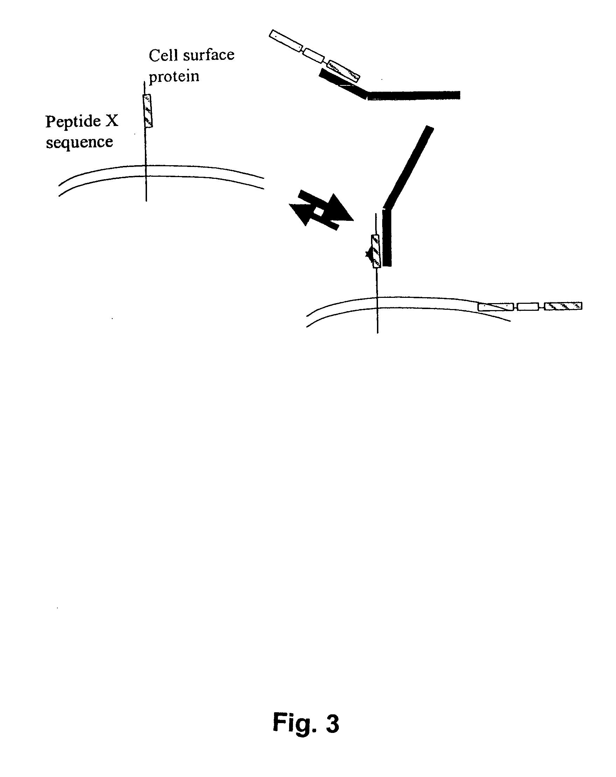

Concomitantly, and as elegantly proven in examples 4 and 12, the present invention relates to a method for designing a selCPP, based on three basic functions: 1) selective cleavage (and thereby activation) by MMP-2 or MMP-MT1, 2) cellular penetration by peptides (CPPs) and 3) killing of nearby, preferably tumour cells or endothelia involved in tumour neovascularisation, by a known cytostatic and/or cytotoxic agent (see Fig.2, 3, 8 and 9).

The present invention thus comprises a selCPP selected from an amino acid sequence contained in table 2 or table 3, and to a combined cell-selective delivery system for a cytostatic and/or cytotoxic agent, comprising an amino acid sequence listed in table 2 or table 3, and a cytostatic and/or cytotoxic agent. See SEQ.ID.NO. 3-10.

**Table 2. selCPPs based on MMP-2 (gelatinase-A) cleavage specificity:**

| **Name** | **Sequence** | **MMP site** | **Penetration** | **Comment:** |
|---|---|---|---|---|
| YTA-2 | YTAIAWVKAFIRKL RK | SGESLAY-YTA | +++ | stain also nuclear membrane Fig.2 |
| | (SEQ ID NO: 3) | (SEQ ID NO: 11) | | |
| YTA-2ps* | SGESLAY-YTAIAWVKAFIRKL RK | SGESLAY-YTA (SEQ ID NO: 11) | + | bind the plasma membrane Fig.3 |
| | (SEQ ID NO: 4) | | | |

| | | | | |
|---|---|---|---|---|
| *ps stands for proteinase cleavage site | | | | |

**Table 3. selCPPs based on MMP-MT1 cleavage specificity**

| Name | Sequence | MMP site |
|---|---|---|
| LRSW-1 | LRSWVISRSIRKAA | GPLG-LRSW |
| | (SEQ ID NO: 5) | (SEQ ID NO: 12) |
| LRSW-2 | LRSWIRRLIKAWKS | GPLG-LRSW |
| | (SEQ ID NO: 6) | (SEQ ID NO: 12) |
| LRSW-3 | LRSWRVIIRNGQR | GPLG-LRSW |
| | (SEQ ID NO: 7) | (SEQ ID NO: 12) |

The LRSW-1 ps, LRSW-2 ps and LRSW-3 ps have the SEQ ID NOs: 8, 9 and 10, respectively.
Consequently, the present invention relates also to a cell-selective delivery system for a cytostatic and/or cytotoxic agent, comprising a) a cell-penetrating peptide and/or a non-peptide analogue thereof comprising a protease consensus site for a protease specifically overexpressed in a target cell and c) a cytostatic and/or cytotoxic agent, wherein said cell-selective delivery system additionally comprises an inactivation sequence repressing the activity of said cell-penetrating peptide, and which is cleaved by said protease specifically overexpressed in the target cell upon introducing said cell-selective delivery system in the near vicinity of said target cell.

In a preferred embodiment of said cell-selective delivery system, as described above, said cell-penetrating peptide comprised in said cell-selective delivery system enhances the average rate of cellular uptake of said cytostatic and/or cytotoxic agent into said selective cell per cell by a factor of at least 1.5 compared to the average rate of cellular uptake into said cell of a cell-selective delivery system comprising only components a) and c), or to the average rate of cellular uptake of component c) alone of said cell.

In another, equally preferred embodiment of said cell-selective delivery system, as described above, said cell-penetrating peptide comprised in said cell-selective delivery system enhances the average rate of cellular uptake of said cytostatic and/or cytotoxic agent into said selective cell per cell by a factor of at least 1.5, such as at least 2, 3, 4, 5, 6, 7, 8, 9, 10, 15, 20, 25, 30, 35, 40, 50, 60, 70, 80, 90, 100, 1000 or 10 000, compared to the average rate of cellular uptake into said cell of a cell-selective delivery system comprising only components a) and c), or to the average rate of cellular uptake of component c) alone of said cell.

In another equally preferred embodiment of said cell-selective delivery system as described above, said overexpressed protease is a Zn²⁺metallo endopeptidase selected from the group consisting of MMP-1, MMP-2, and MMP-MT1.

In yet another embodiment, said overexpressed protease is selected from the group consisting of bacterial surface proteases and viral enzymes.

A cell-selective delivery system as described above can of course be used for the manufacture of a pharmaceutical composition for stopping cellular proliferation of a specific cellular population in a mammal and for treating a patient suffering from a medical condition characterised by uncontrolled cellular growth, such as any oncological disorder or disease, or immunological and/or metabolic hyperfunction.

Yet another aspect of the invention is directed to the use of a cell-selective delivery system according to the invention for the manufacture of a medicament.

The different aspects and embodiments of the invention will now be illustrated by the following examples. It should be understood that the invention is not limited to any specifically mentioned details.

### ABREVIATIONS

- Aβ: beta-amyloid
- AD: Alzheimer's disease
- APP: Amyloid precursor protein
- AT1: angiotensin receptor type AT1
- AT1A: angiotensin receptor subtype AT1A
- AT1B: angiotensin receptor subtype AT1B
- AT2: angiotensin receptor type AT2
- BACE: β-site APP-cleaving enzyme
- Bio: biotin, biotinylated
- BSA: bovine serum albumin
- CPP: cell-penetrating peptide
- CTF: C-terminal fragment
- DCC: N,N'-dicyclohexylcarbodiimide
- DCM: dichloromethane
- DIEA: diisopropylethylamine
- DMF: dimethylformamide
- DNP: dinitrophenyl
- EOFAD: early onset Alzheimer's disease
- FITC: 5-fluorescein isothiocyanate
- Fmoc: 9-fluorenylmethoxycarbonyl
- GOP: IVIAKLKA-amide
- GPCR: G-protein coupled receptor
- GTP: guanosine 5'-triphosphate
- GTPase: guanosine triphosphatase
- GTPγS: guanosine γ-S-5'-triphosphate
- HKR: Hepes-Krebbs-Ringer
- HOBt: N-hydroxybenzotriazole
- HPLC: high performance liquid chromatography
- IDE: insulin degrading enzyme
- LOAD: late onset Alzheimer's disease
- MBHA: 4-methylbenzhydrylamine
- NFT: neurofibrillary tangles
- NICD: notch intracellular domain
- NMP: N-methylpyrrolidone
- NTF: N-terminal fragment
- PAF: paraformaldehyde
- PBS: phosphate buffered saline
- PNA: peptide nucleic acid
- PS: presenilin
- RNA: ribonucleic acid
- SAPP: secretory APP
- TACE: tumour necrosis factor alpha converting enzyme
- *t*-Boc: tert-butyloxycarbonyl
- TBTU: 2-(1H-benzotriazole-1-yl)-1,1,3,3-tetramethyluronium etrafluoroborate
- TFA: trifluoroacetic acid
- TFA: trifluoroacetic acid
- TFMSA: trifluoromethanesulphonic acid

### LEGENDS TO FIGURES

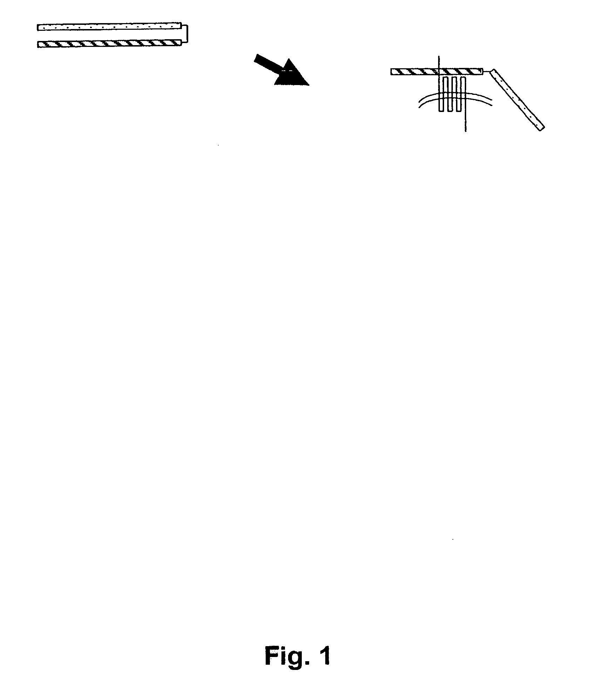

Fig. 1. Intramolecularly constrained selCPP looses its constrain upon recognition event by specific receptor and the internalisation takes place.
Fig. 2. Schematic structure of chimeric selCPP.
Fig. 3. Incubation of non-covalent selCPP-AB complex with the selected cells exposing the epitope sequence. Peptide X leads to competitive interaction of AB with the Peptide X sequence in the cell surface protein.

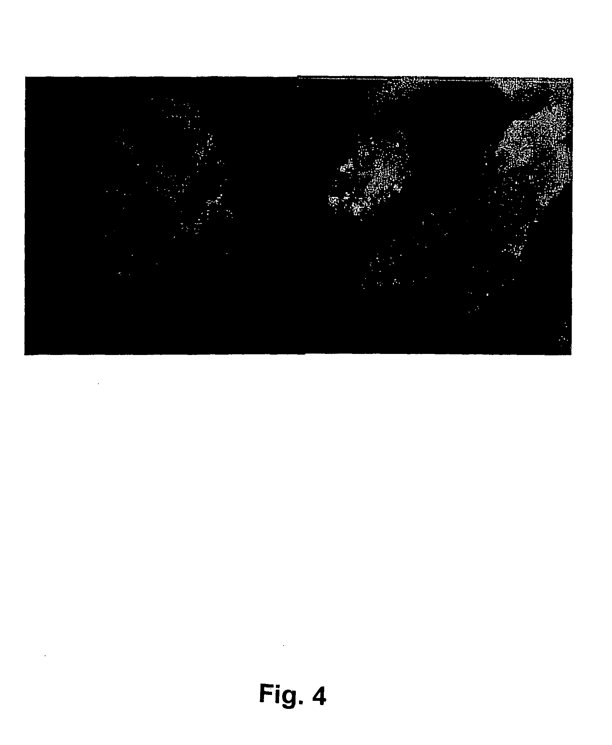

Fig. 4. Example of inactivated selCPP. Internalisation of YTA-2 (A) compared to YTA-2ps (B) in LoVo cells, both biotinylated peptides detected by TRITC-avidin at 37°C.

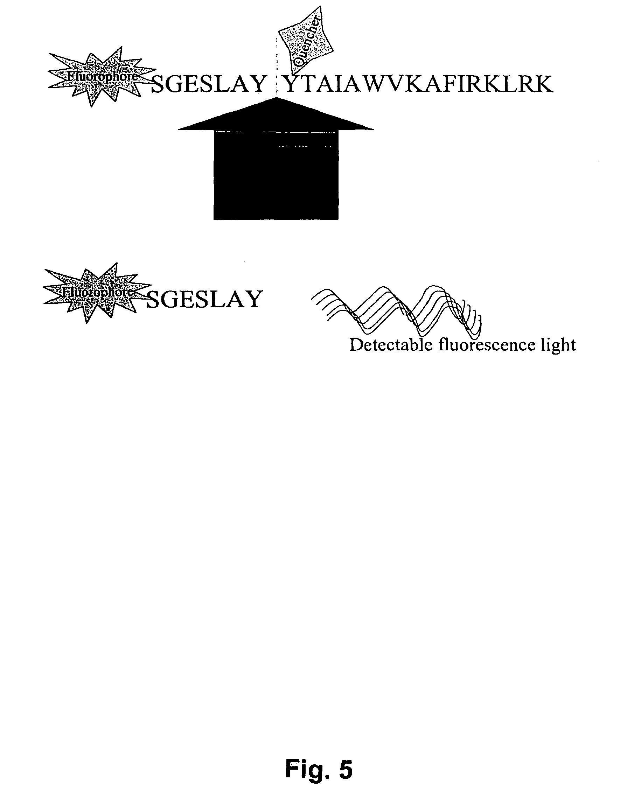

Fig. 5. Example of protease activated seICPP, detection by fluorophore/quencher system. Method of determining the specific cleavage of YTA-2ps of the matrix metallo proteinase-2 (MMP-2).

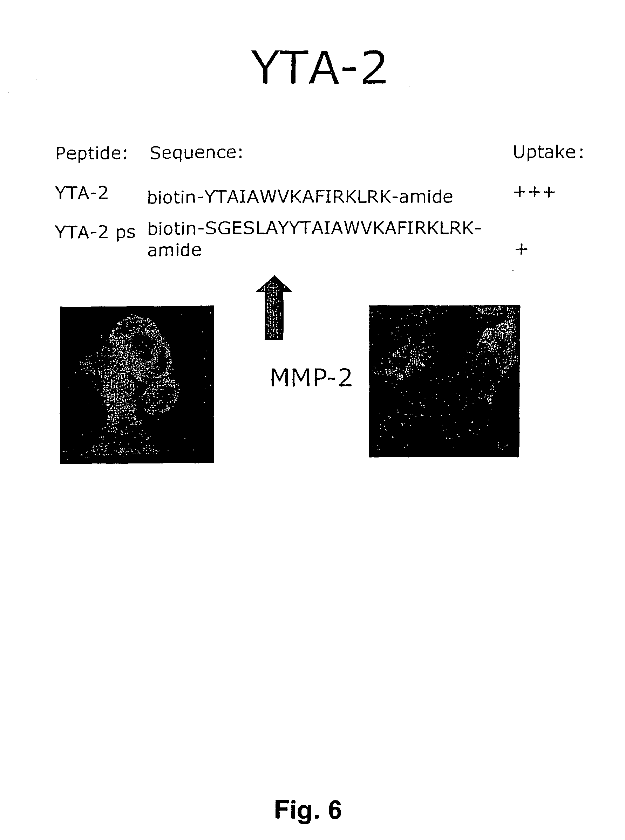

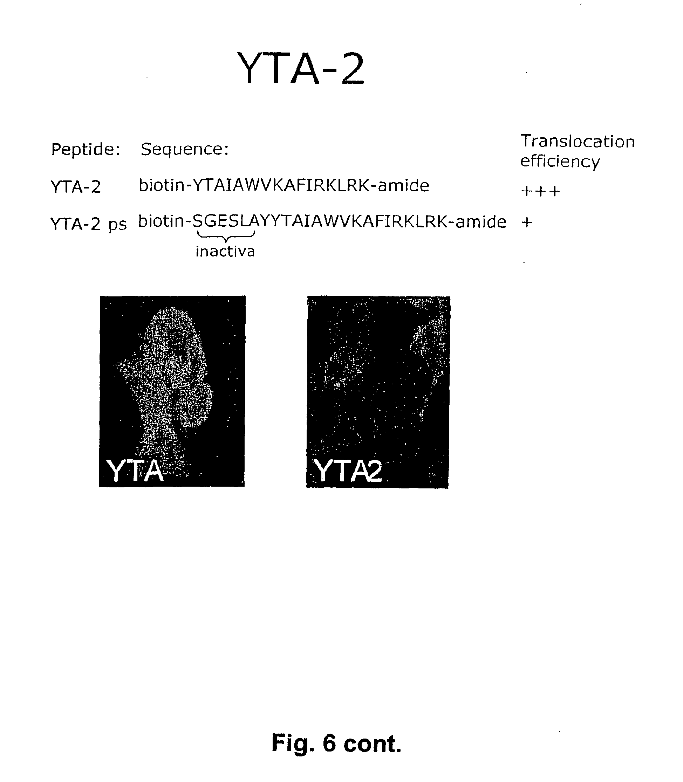

Fig. 6. Translocation of 10µM peptide at 37 °C in LoVo cells (human colon cancer).
Fig. 7. Another example of an inactivated selCPP. PenMMP14 uptake in Bowes (A) and B)) and Caco-2 (C) and D)). The left column is uptake of fluorescein labelled peptide (A) and C)) and the left with coumarine label (B) and D)).
Fig. 8. Scheme of selCPP activation by matix metalloproteases (a) representative for any tissue/organ/cell specific protease, leading to tissue-(tumour) selective uptake (b).
Fig. 9. Scheme of selCPP activation by matix metalloproteases(a) leading to tissue-(tumour) selective uptake (b).
Fig. 10. Internalization of biotinylated YTA-2 in human colon adenocarcinoma, LoVo cells.A) Deteceted with streptavidin-FITC B) comparison nuclear staining with Hoechst. B)
Fig. 11. lnternalisation of YTA-2 (A) compared to YTA-2ps (B) in LoVo cells, both biotynilated peptides detected by TRITC-avidin at 37°C. C) and D) YTA-2 in Caco-2 cells detected with streptavidin TRITC and nuclear stain.

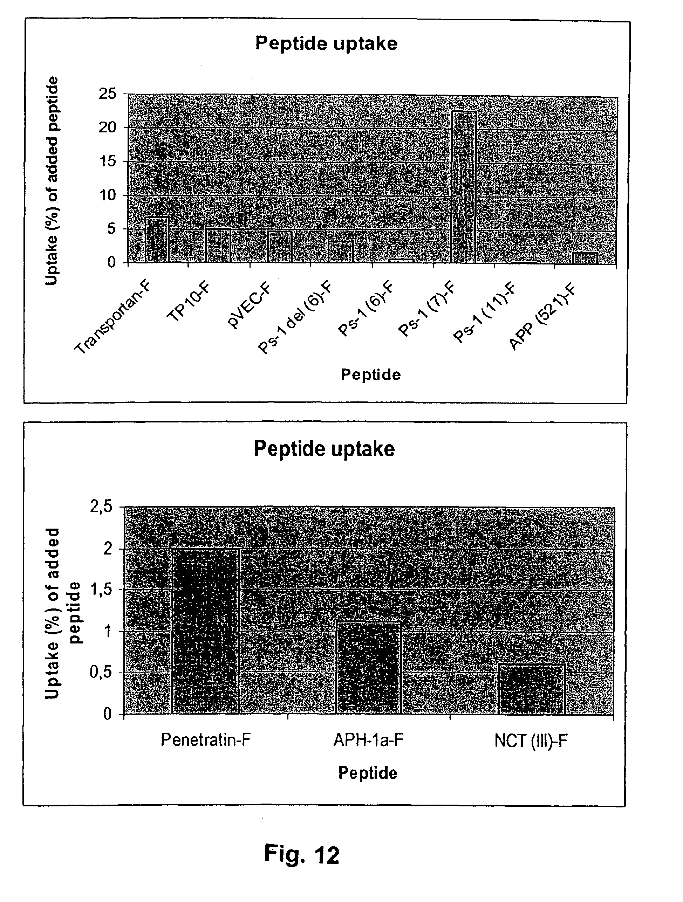

Fig. 12. Uptake of fluorescently labeled peptide (F). Figures are shown in % uptake of added peptide.
Fig. 13. Penetration of Apa-γGlu-Gly-Evo165 into cultured human epidermal keratinocytes, imunofluorescent detection.
Fig. 14. Effects of different MTX-pVEC conjugates on Bowes cell viability (assayed using Cell-TiterGlo^{™}). Exposure for 24h in 10% FBS-MEM.
Fig. 15. Effect of Apa-γGlu-Gly-Evo165 on Bowes cell viability (assayed using Cell-TiterGlo^{™}).
Fig. 16. Effects of MTX, Apa-γGlu-Gly-YTA2 and YTA2 on Bowes cell viability (assayed using Cell-TiterGlo^{™}).
Fig. 17. Effects of MTX, Apa-γGlu-Gly-YTA2 and YTA2 on K562 cell viability (assayed using Cell-TiterGlo^{™}). Exposure for 2 days in 7.5% FBS-RPMI.
Fig. 18. siRNA linked with disulfide bonds to Tp10-PNA, schematic view of construct and theoretical mechanism of action in the cell.
Fig. 19. Uptake of 1 µM peptide-DNA construct, 30 min 37°C in Bowes cells, 24 well plate.
Fig.20. Example of cellular penetration by well studied CPPs. Peptide internalisation in live Caco-2 cells of 1 µM Fluo-peptide.
Fig.21. Protein internalisation by YTA-2 of Fluo-Streptavidin 37°C and T/E, showing the delivery property of a selCPP.
Fig.22. Protein internalisation by YTA-2 of Fluo-Streptavidin 4°C and T/E, showing the delivery property and temperature independency of a selCPP.

### EXPERIMENTAL SECTION

### Example 1

### Peptide synthesis (describing default method of the experiments below if not indicated otherwise)

Peptides were synthesized in a stepwise manner in a 0.1 mmol scale on a peptide synthesizer (Applied Biosystems model 431 A, USA) using t-Boc strategy of solid-phase peptide synthesis. tert-Butyloxycarbonyl amino acids (Bachem, Bubendorf, Switzerland) were coupled as hydroxybenzotriazole (HOBt) esters to a p-methylbenzylhydrylamine (MBHA) resin (Bachem, Bubendorf, Switzerland) to obtain C-terminally amidated peptide. Biotin was coupled manually to the N-terminus by adding a threefold excess of HOBt and o-benzotriazole-1-yl-N, N, N', N'-tetramethyluronium tetrafluorborate (TBTU) activated biotin (Chemicon, Stockholm, Sweden) in DMF to the peptidyl-resin. The peptide was finally cleaved from the resin with liquid HF at 0°C for 30 min in the presence of p-cresol. The purity of the peptide was >98% as demonstrated by HPLC on an analytical Nucleosil 120-3 C-18 RP-HPLC column (0.4 x 10 cm) and the correct molecular mass was obtained by using a plasma desorption mass spectrometer (Bioion 20, Applied Biosystems, USA) or MALDI-TOF (Vaager STR-E, Applied Biosystems, USA), as described in (Langel, U., Land, T. & Bartfai, T. Design of chimeric peptide ligands to galanin receptors and substance P receptors. Int J Pept Protein Res 39, 516-22. (1992)).

### Cell culture (describing defaultmethod of the experiments below if not indicated otherwise)

Murine fibroblasts C3H 10T1/2, mouse neuroblastoma N2A cells and COS-7 cells were grown in 10 cm petri dishes in Dulbecco's Modified Eagle's Media (DMEM) supplemented with 10% fetal calf serum (FCS), 2mM L-Glutamine, 100 U/ml penicillin and 0.1 mg/ml streptomycin at 37°C in a 5% CO₂ atmosphere. The cells were seeded and replated every fifth day. COS-7 and 10T1/2 cells were trypsinized when seeded while the N2A cells were suspended by mechanical force by adding media to the cells. Before starting the experiments, the cells were grown to confluency and then seeded and diluted two times in media before adding to 24-well plates (approximatly 60 000 cells/well).

### High throughput screening (HTS) of cargo delivery efficiency.

By using CPP-S-S-cargo constructs where the cargo is labelled with the 2-aminobenzoic acid fluorophore and the CPP with the 2-nitrotyrosine quencher, it is possible to monitor, in real time, the intracellular degradation of the disulfide bond resulting from the reducing intracellular milieu, and hence the cellular uptake of the constructs by increase in apparent fluorescence.

### Peptide uptake and outflow studies in cells in suspension or attached

Cells were detached with trypsin (Invitrogen, Sweden), dissolved in culture media and centrifuged (1000 xg for 10 min at RT). The cells were resuspended, counted and aliqoted in HKR on ice, 300 000 cells /tube. Abz-labelled peptide was incubated for 15 and 30 min together with the cells in suspension, on a shaking 37 °C water bath. To stop the uptake or outflow, trypsin solution was added for 3 min. The cells were spun down at 1000x g for 10 min at 4°C. The pellets were resuspended in HKR for fluorescence detection, or for the outflow samples, incubated again with peptide-free HKR. Fluorescence was read at 320/420 nm on a Spectramax Gemini XS (Molecular Devices, CA). The intracellular concentrations were calculated from a standard curve of Abz-labelled peptides. The average cell volume of Caco-2 cells was determined by using a Coulter 256 channelizer (Coulter Electronics Ltd. CA). Additionally, a varianty of the same assay was applied: the cells were seeded in a 24well plate at a density of 100 000 cells /well, the day before. Cells are incubated at 5µM peptide concentration for 30 min at 37° C. Trypsin treated, washed and lysed in hydrochloric acid. The fluorescence was measured as described for the suspension assay.

### Materials and methods

### Cells

Human melanoma cells Bowes, were cultured in MEM using standard cell culturing techniques, and seeded at density of 100 000cells/well in 24 well-plates the day before experiments were conducted.

### Results and discussion

### Table 4. Examples demonstrating the quantitative measurements for the ability of various

CPPs to enter Bowes cells when added for 30 min at 37° C at 5µM concentration.

| **Fluoresceinyl peptide** | **% of added fluorescence in cell lysate** |
|---|---|
| | 10.5 |

| **Transportan** | |
|---|---|
| ***p*VEC** | 4.6 |
| **YTA-2ps** | 3.5 |
| **LRSW-3** | 1.9 |
| **TP10** | 1.8 |
| **LRSW-1** | 1.4 |

Table 4 Shows data from the preferred method to verify that the peptide is in fact a cell-penetrating peptide: Peptide uptake and outflow studies in cells in suspension or attached (see below). Approximately 50 peptides have been screened so far. For negative control the non-membrane permeable sugar polymer fluoresceinyl Dextran is added to the cells in the same manner, the percentage found in cell lysate of Dextran is always < 0.5 %. Note that YTA-2ps, LRSW-1 and LRSW-3 are examples of *de novo* designed artificial peptides.

### Example 2

### Method of determining the specific cleavage of YTA-2ps of the matrix metallo proteinase-2 (MMP-2):

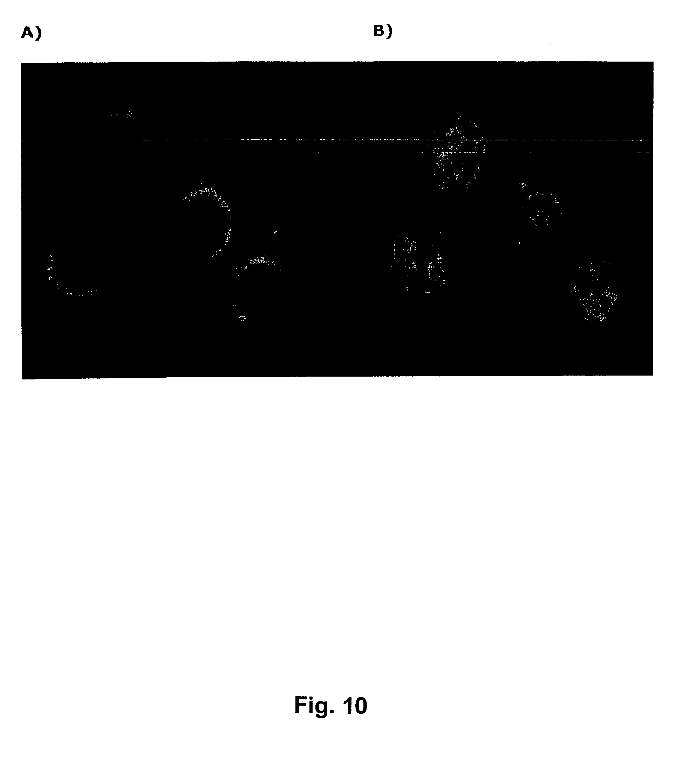

As illustrated above and in Fig. 10, the inventors have been able to show that the CPP-part of a selective CPP (YTA-2) can efficiently enter cells both at 37° and 4° C (data not shown). In addition, the "inactivator" made the peptide less active in translocation over the cell membrane. The next step in the development of this technique is to check the specific cleavage of YTA-2ps by active MMP-2. It is performed by a fluorescence/quencher assay, wherein the MMP-2 substrate YTA-2ps upon cleavage increases in fluorescence intensity. The correct cleavage is further checked by mass spectrometry.

### Example 3

### Materials and Methods

### Synthesis and purification of TP-10 and YTA-2

The peptides were synthesized in a stepwise manner on an Perkin Elmer/ Applied Biosystem Model 431 A peptide synthesizer, using *t*-Boc strategy according to protocol described previously (Langel, Land et al. 1992). Cysteine or glutamic acid was coupled manually. TP10 sequence is given in Pooga, 1998, FASEB J.(SEQ ID NO: 2).YTA-2 sequence is given in table 2 and listed as SEQ.ID.NO: 3. Prior conjugating peptides to PEI, they were purified on a reversed phase HPLC (Gynkotek) C18 column with AcN/H₂O gradient, and analyzed using a MALDI-TOF Mass spectrometer (Applied Biosystems model Voyager STR). The mass values obtained matched calculated values.

### Cell culture

Murine fibroblasts C3H 10T1/2, mouse neuroblastoma N2A cells and COS-7 cells were grown in 10 cm petri dishes in Dulbecco's Modified Eagle's Media (DMEM) supplemented with 10% fetal calf serum (FCS), 2mM L-Glutamine, 100 U/ml penicillin and 0.1 mg/ml streptomycin at 37°C in a 5% CO₂ atmosphere. The cells were seeded and replated every fifth day. COS-7 and 10T1/2 cells were trypsinized when seeded while the N2A cells were suspended by mechanical force by adding media to the cells. Before starting the experiments, the cells were grown to confluency and then seeded and diluted two times in media before adding to 24-well plates (approximatly 60 000 cells/well).

### Example 4

### selCPP

### Introduction

Matrix metallo proteases (MMPs) are Zn²⁺ metallo endopeptidases. The family contains both membrane bound and secreted members of which both catalyse the breakdown of proteins located either on the cell's plasma membrane or within the extracellular matrix (ECM) (Sternlicht-01).

Because MMPs can degrade the ECM, MMP's influence cell migration, remodelling, and inflammatory responses. However, these enzymes may also be involved in the underlying causes of invasive and inflammatory diseases, including cancer, rheumatoid arthritis, multiple sclerosis and bacterial meningitis (Leppert-01).

One characteristic of invasive processes like metastasis and angiogenesis is the degradation of the ECM and basal membranes, which are normally physical barriers to cell migration. MMPs have been linked to the invasive and metastatic behaviour of a wide variety of malignancies, and these enzymes are generally overexpressed in a variety of tumours (Sternlicht-01, Rozanov-01). The number of different MMPs have been found to increase with tumour progression (Hoekstra-01) and correlate to the invasive capacity of certain tumours (Hornebeck 02). Furthermore, expression of MMPs have been correlated to the number of metastatic growth in transgenic mice (Strenlicht-01).

Membrane type MMPs (MT-MMP) such as MMP-MT1 have been strongly implicated in oncogenesis. These enzymes localise to the invasive fronts. The soluble MMPs 1-3 and 9 have also been implicated as agonists of tumourigenesis (Rozanov-01 and Nabeshima-02). MMP-MT1 is also upregulated during endothelial cell induction and migration during angiogensis (Galvez-01). In addition, MMP-MT1, or MMP14 as it is also called, is involved in the activation of proMMP-2 by its "shedase" activity, thereby releasing active MMP-2 at the inasive front (Sounni-02). MMP-2 appears to have an important role in tumour angiogensis (Chen-01).

### seICPPs

The concept of selective CPPs are based on the tissue specificity of MMPs enzyme activity. One approach is to use endocytotic uptake for specificity and activation of the CPP by conjugating it to any receptor ligand, such as galanin.

**Table 5. Sequences of sel CPPs**

| **Name** | **Sequence** | **Uptake** | **In Cells** |
|---|---|---|---|
| **YTA-2 SelCPP1** | biotin-YTAIAWVKAFIRKLRK-amide (SEQ lD NO: 3) | +++ | Lovo, bEnd, Caco |
| **YTA2-ps** | biotin-SGESLAY-YTAIAWVKAFIRKLRK-amide (SEQ ID NO: 4) | + | Lovo, bEnd, Caco |
| **LRSW-1** | LRSWVISRSIRKAA-amide (SEQ ID NO: 5) | nd | synthesis |
| **LRSW-2** | LRSWIRRLIKAWKS-amide (SEQ ID NO: 6) | nd | synthesis |
| **LRSW-3** | LRSWRVIIRNGQR-amide (SEQ ID NO: 7) | nd | synthesis |
| **SelCPP2** (Fig.7) | coum-DEEQERSEN-IRQIKIWFQNRRMKWK*K-amide (SEQ ID NO: 13) | ++ | bEnd, Caco, SHS5Y |

| | | | |
|---|---|---|---|
| * fluoresceinlabelled (IRQIKIWFQNRRMKWKK = SEQ ID NO: 20.) | | | |

### MMP activated seICPP

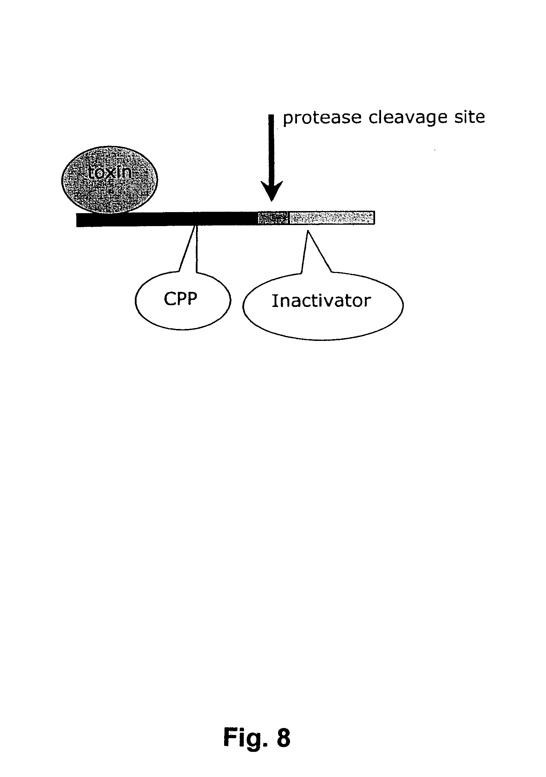

A typical seICPP (Fig.8) is made up of three parts, the transporter (CPP), the specific protease site and the inactivator. The inactivator is added to inactivate the CPP, so that it cannot enter cells before the inactivator is cleaved off. The CPP carries a toxin, for example a known non-permeable cytostatic and/or cytotoxic agent. For preferred embodiments of sequences and labelling of the peptides see table 5.

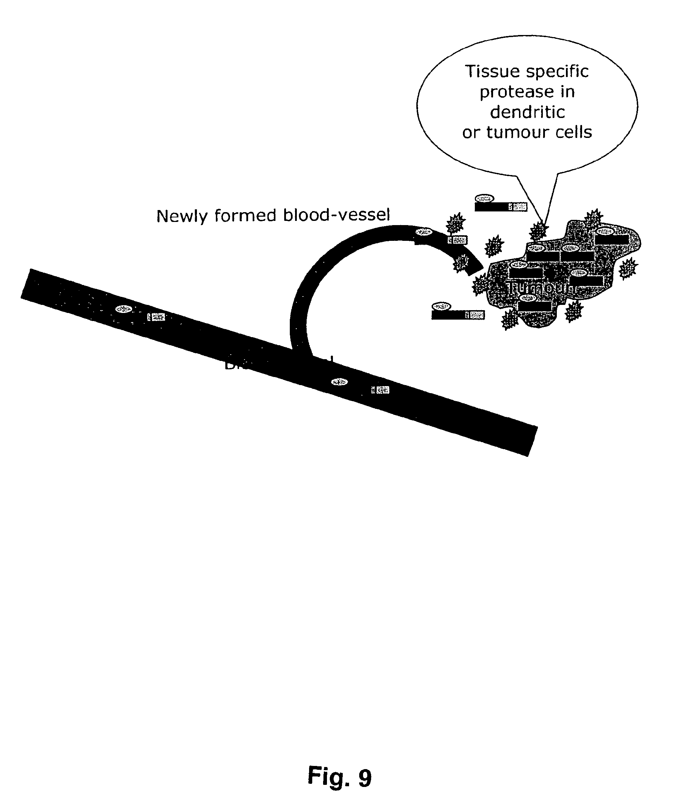

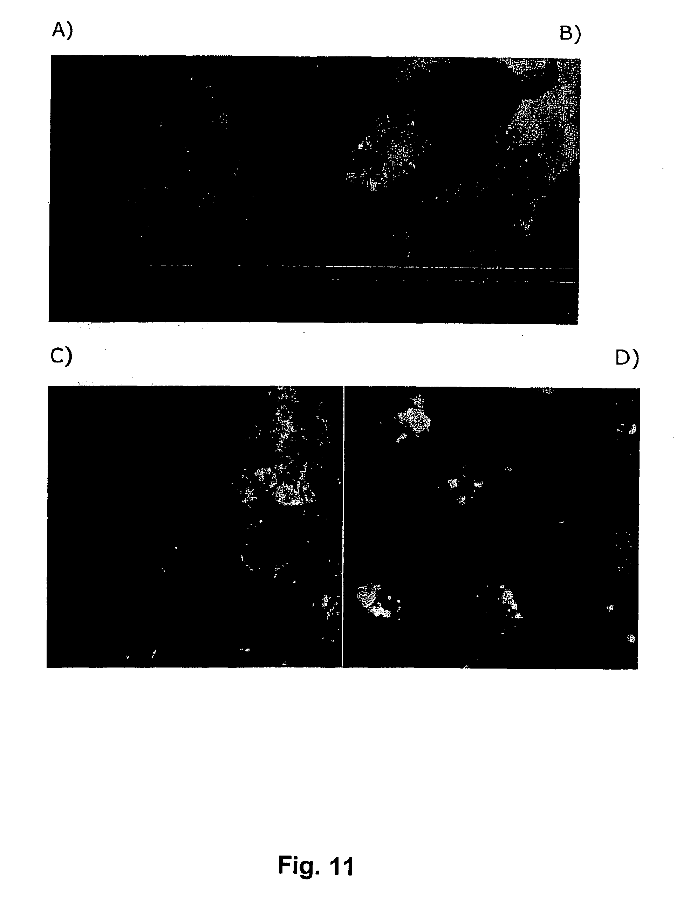

The idea is based on three basic functions: a) selective cleavage (and thereby activation) by MMP-2 or MMP-MT1, b) cellular penetration by peptide (CPP) carrying the toxin and thereby c) killing of nearby, preferably tumour cells or endothelia involved in tumour neovascularisation (a schematic view is given in Fig.8).
As an illustrative example to prove the above concept, the present inventors have successfully been able to show that the CPP-part of the selective CPP (YTA-2) can efficiently enter cells both at 37° (Fig.10 and 11) and 4° C (data not shown). In addition, the "inactivator", see Fig.9, renders the peptide less active in translocation over the cell membrane. The correct cleavage of YTA-2 by MMP2 has also been confirmed by mass spectrometry (data not shown). The attachment and efficiency of cytostatic and/or cytotoxic agent (MTX) to the CPP-part, see example 5.

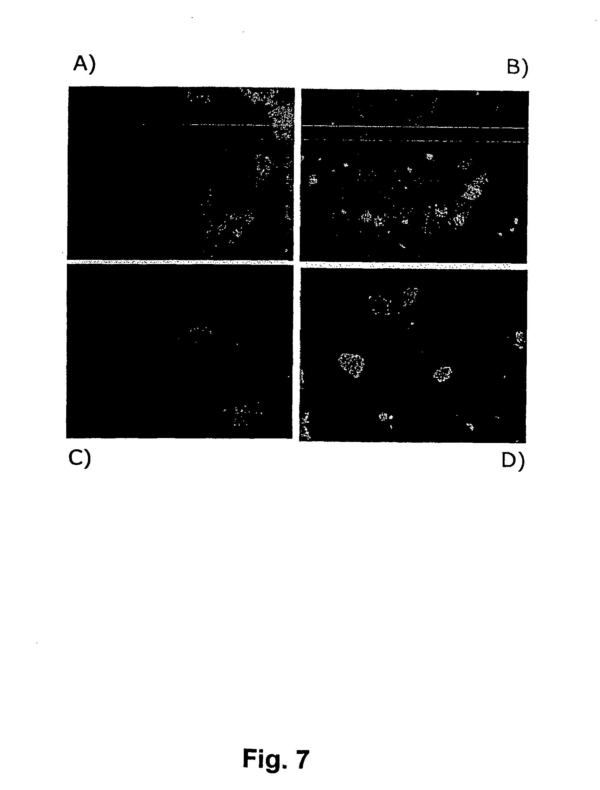

There are four new sequences for cleavage of MMP 14 (or MMP-MT1): the LRSW1-3 and penMMP14. The latter has been shown to be taken up in cells expressing MMP14 (Bowes and Caco-2) see Fig.7.

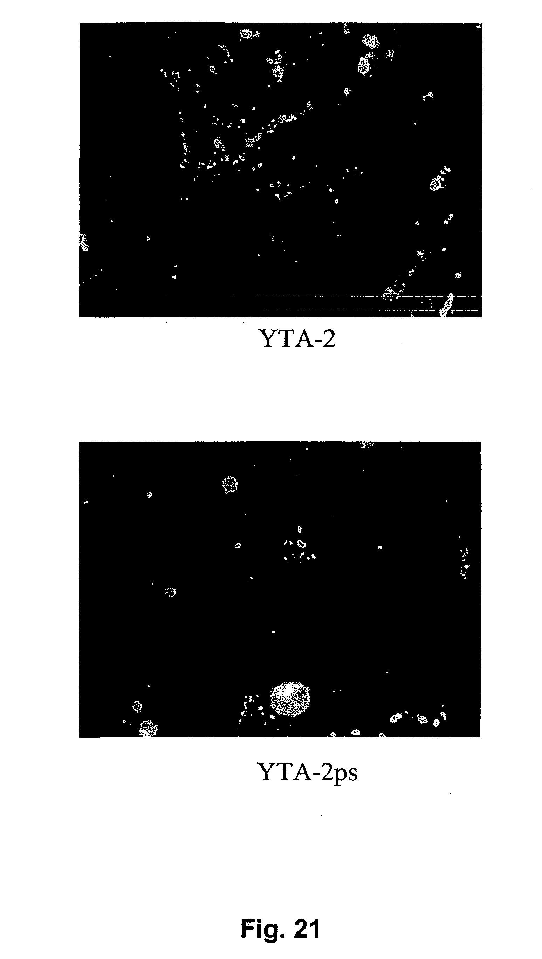

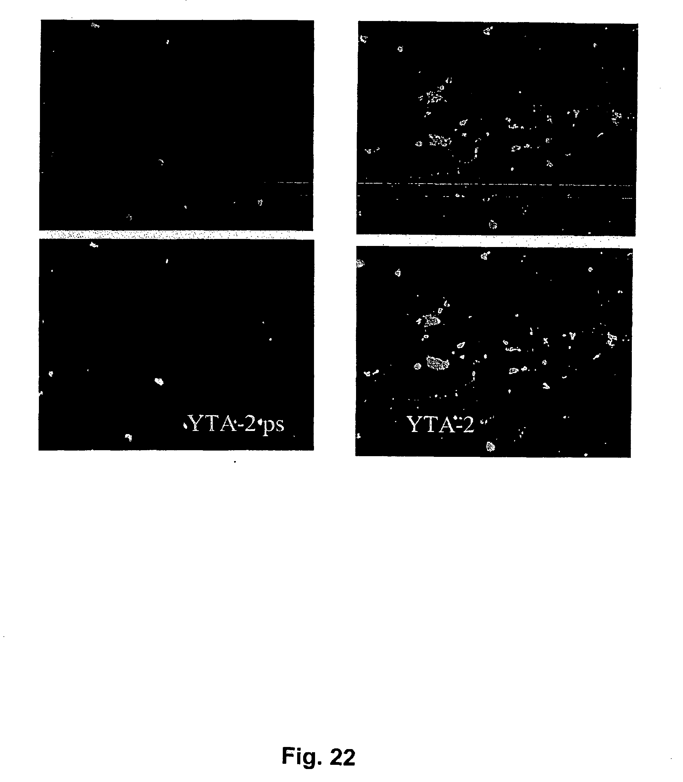

Additionally, the 67kDa protein fluorescein labelled streptavidin was internalised when linked to biotinylated YTA-2 (see figures 21 and 22). This shows that YTA-2 can transport large cargo molecules into cells.

**Table 6. Exression of MMP2 and MMP14 in cell lines in the lab.**

| Cells: | MMP2 expression (Experiment): | MMP14 expression (Literature) |
|---|---|---|
| Caco-2 | - | + |
| Lovo | - | + |
| SHS5Y | + (released inactive) | not determined |
| Bowes | + (released inactive) | + |
| PC 12 | + (released inactive) | not determined |
| Rinm5F | - | not determined |

All the seICPP tested for uptake so far are confirmed with mass spectrometry. Furthermore the stability of the peptides in cell culture media and in cell lysate are determined. In addition, the selectivity of the peptides are tested in *in vitro* cell assays, for example with cells expressing the selectivity protease mixed with cells that do not express the protease for activation of the CPP part (Table 6.)

### Example 5

### Example of intracellular drug delivery: conjugate of methotrexate and cell-penetrating peptide (MTX-CPP)

### Introduction

Methotrexate (MTX) is a cytotoxic drug, which was developed for the treatment of malignancies but is now also used to cure autoimmune diseases, such as psoriasis. MTX is a folate antagonist (Formule 1) and enters the cell via folate transporters. The targets of MTX are folate-requiring enzymes.
The usage of MTX in the treatment of psoriasis is hampered due to its side-effects at the systemic administration, most serious is hepatotoxicity. Therefore, a topic administration formulation of MTX is highly desirable.

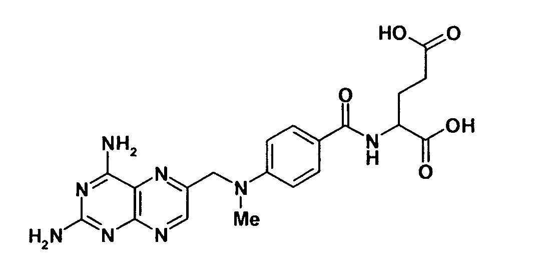

### Formule 1. The structure of MTX.

In the biological fluids, MTX is present as negatively charged molecule. Therefore, it can cross the cell membrane only via folate transporters. The present invention for the first time reveals the means to produce an MTX-CPP conjugate which:
1) penetrates readily and receptor/carrier-independently into cells and into the skin
2) hampers the keratinocyte hyperproliferation, a hallmark of psoriasis
3) does not have unwanted side effects (such as histamine release).

All the MTX-CPP conjugates are synthesised using solid phase peptide synthesis strategy. This is possible due to two reasons:
1) MTX (CAS no 59-05-2) can be divided into two structural units: 4-deoxy-4-amino-N10-methylpteroic acid (Apa, CAS no 19741-14-1) and γ-glutamate
2) MTX is relatively unsensitive to substitutions on its γ-carboxyl group

**Table 1. MTX-CPP conjugates**

| |
|---|
| Apa-γGlu-Gly-CPP |
| Apa-(γGlu)2-5-Gly-CPP |
| Apa-Cys-S-S-Cys-CPP |

As shown in Table 1, several MTX-CPP conjugates are designed. It is expected that the CPP part of the conjugate is degraded in the cell.

### Solid phase synthesis of MTX-CPP conjugates

Fmoc-chemistry was used due to MTX not being stable under standard cleavage conditions for Boc-chemistry (hydrogen fluoride at 0°C for 30 min). The couplings of Fmoc-Glu-OtBu and Apa were performed using the standard coupling method (HOBt/TBTU). The conjugates were cleaved from the resing using the Reagent K (82.5% TFA: 5% phenol: 5% thioanisole:2.5% 1,2-ethanedithiol).

### Cell cultures

All cells were cultured at 37°C in 5% CO₂. The plastic labware was from Corning Inc. (Acton, MA). Neonatal human epidermal keratinocytes (HEKn), all growth media components and trypsination reagents necessary for their propagation were obtained from Cascade Biologics^{™} (Portland, OR). HEKn cells were cultured in Epilife® Medium supplemented with human keratinocyte growth supplement kit (HKGS) and penicillin, streptomycin and amphotericin B. HEKn cells were splitted once a week, seeding 300 000 cells per T75. Growth medium was changed every 1-2 days.

Bowes human melanoma cells were obtained from American Type Culture Collection (Manassas, VA). Bowes cells were cultured in Minimal Essential Medium with Earle*'*s salts complemented with Glutamax-I, non-essential amino acids, sodium pyruvate, penicillin, streptomycin (referred on figures as "MEM") and 10% foetal bovine serum (FBS). All media components for Bowes cells were from Invitrogen Corporation (Paisley, UK). Trypsin/EDTA was from PAA laboratories GmbH (Linz, Austria). Cells were subcultured once a week, seeding 200 000 cells per T75. For the viability assays, 50 000 cells (in 300 µl) were seeded per a well in a 24-well-plate a day before the experiment.

### K562 human erythroleukemia cells were obtained from American Type Culture Collection (Manassas, VA). K562 cells were cultured in RPMI-1640 medium complemented with

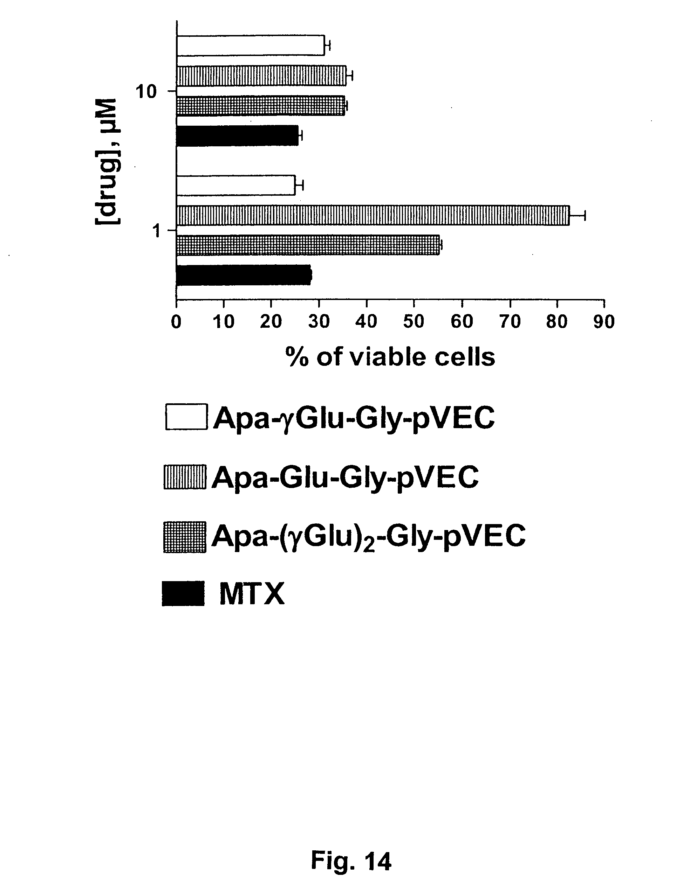

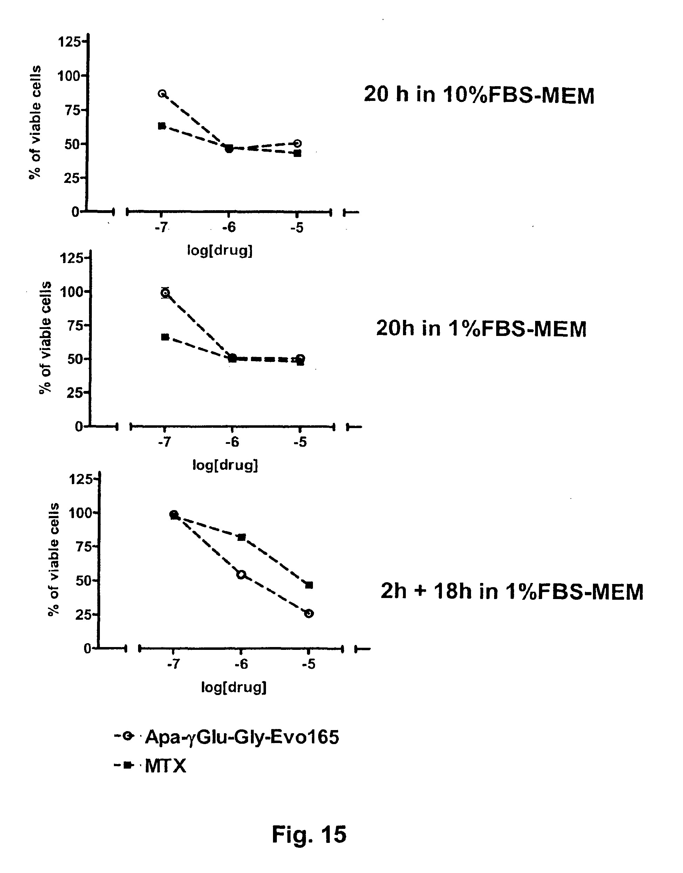

Glutamax-I, penicillin, streptomycin (referred in Fig.14-17 as "RPMI") and foetal bovine serum (7.5%). All media components for K562 cells were from Invitrogen Corporation (Paisley, UK). Trypsin/EDTA was from PAA laboratories GmbH (Linz, Austria). Cells were subcultured every 2^{nd} or 3^{rd} day (when cell density had reached 1 000 000 cells/ml), seeding 100 000 cells per ml. For the viability assays, 30 000 cells in 300 µl were seeded per a well in a 24-well-plate.

### Cell viability measurements

The stock solutions of the conjugates (1 mM) were prepared in sterile water and the stock solution of MTX in 10% DMSO in water. For the exposure, the drugs were diluted in the exposure medium (serum-free OPTIMEM or 1%FBS in MEM or 10%FBS in MEM or 7.5% FBS in RPMI) to the desired concentrations (in the case of Bowes cells) or to 10x final concentration (in the case of K562 cells). 300 µl (for Bowes) or 30 µl (for K562) of the respective exposure mix was used per well. In the case of some experiments with Bowes cells, the exposure mix was removed after 2 h and replaced with prewarmed drug-free exposure medium. After 1-2 days the cell viability was assayed using CellTiter-Glo^{™} Luminescent Cell Viability Assay (Promega, Madison, WI). The plates were equilibrated to the room temperature (approximately 30 min). 300 µl of CellTiter-Glo^{™} Reagent was added to each well and incubated for 10 min. Then 300 µl was transferred to a white polystyrene FluoroNunc^{™} plate (Nunc A/S, Roskilde, Denmark) and luminescence was recorded at dual-scanning microplate spectrofluorometer (SPECTRAmax® GEMINI XS, Molecular Devices, Sunnyvale, CA).

### Results

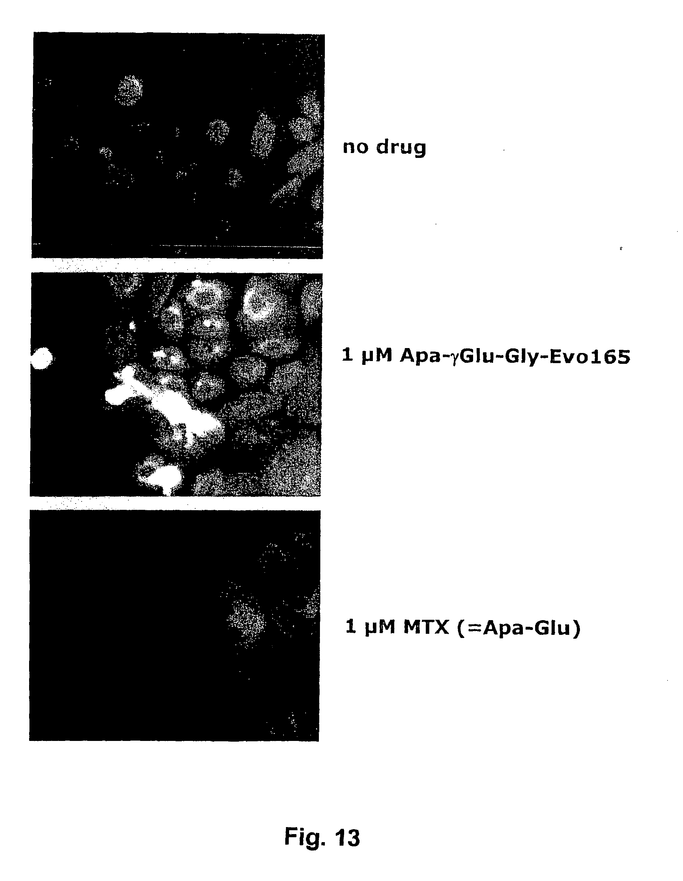

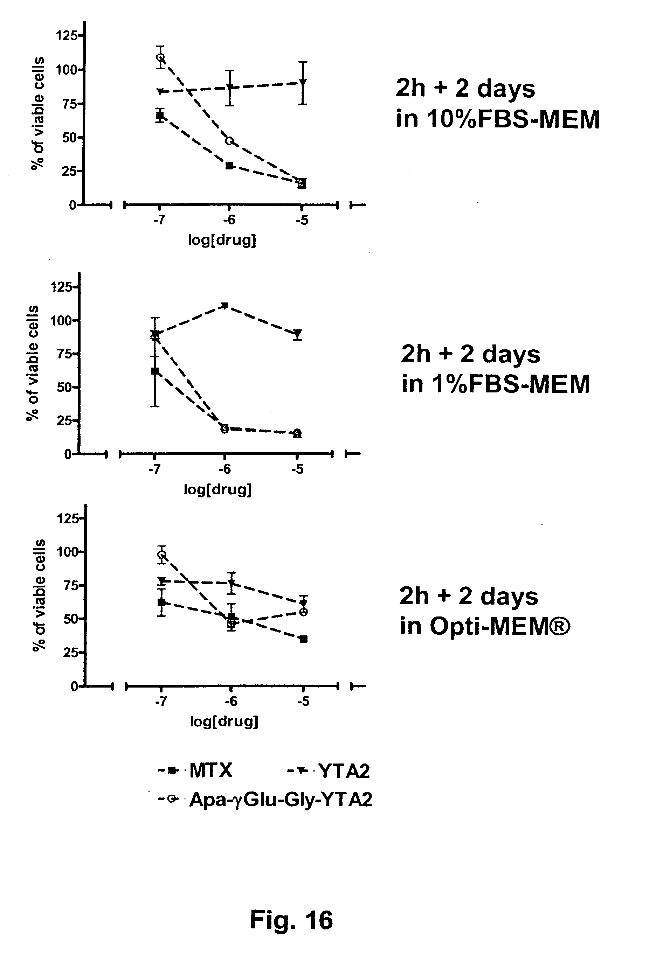

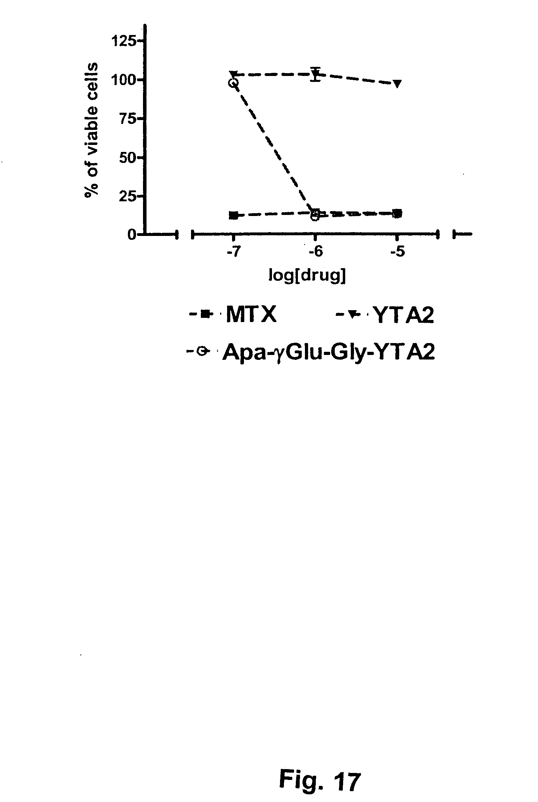

The results obtained so far indicate clearly that conjugation of an MTX to a CPP does not abolish the cell-penetrating nature of a CPP (as exemplified in Fig. 13, wherein Apa-γGlu-Gly-Evo165 is shown to penetrate into human epidermal keratinocytes) (the amino acid sequence of Evo165 appears from SEQ ID NO: 14 (31925) and the variants Evo165b - Evo165f appear from SEQ ID NOs: 15 - 19 (31926 - 31930). Also, the inventors find that the conjugation of a CPP to MTX does not abolish the toxic effect of MTX. Note that the γ-carboxyl group of MTX is more suitable for the conjugation to CPP than the a-carboxyl group of MTX (as seen in Fig. 14, wherein Apa-γGlu-Gly-pVEC is demonstrated to be more toxic than Apa-Glu-Gly-pVEC). What is more, a variety of different CPP-s can be used in the MTX-CPP conjugate as exemplified by all tested conjugates so far: Apa-γGlu-Gly-pVEC in Figure 14, Apa-γGlu-Gly-Evo165 in Figure 16 and Apa-γGlu-Gly-YTA2 in Figure 16 and 17).

### Example 6

### Improvement of siRNA uptake using CPP

In resent years, small interfering RNA (siRNA) have gained a lot of attention for their highly sensitive ability to regulate gene expression in mammalian cells. siRNA are short strands (about 20bp) of double stranded RNA that induce specific cleavage of their complementary mRNA through activation of the RNA-induced silencing complex (RISC). The RNA-induced silencing is an endogenous mechanism, but synthetically synthesized siRNA's have been shown to have the same effect both *in vitro* and *in vivo.*

Unfortunately, a typical problem when using siRNA is the low yield of uptake in the cell. By coupling cell-penetrating peptides (CPP) according to the present invention to synthetically synthesized siRNA the inventors were able to improve the cellular uptake both *in vitro* and *in vivo.*

A siRNA was designed against the galanin receptor-1 (GALR-1) mRNA to which a CPP Transportan10 (Tp10) was coupled via a disulfide linker. The properties of the CPP were shown to increase the cellular uptake of the siRNA, thus rendering it more suitable of pharmaceutical usage. A schematic view of the mechanism of action is given in Fig. 21.

### Material and Methods:

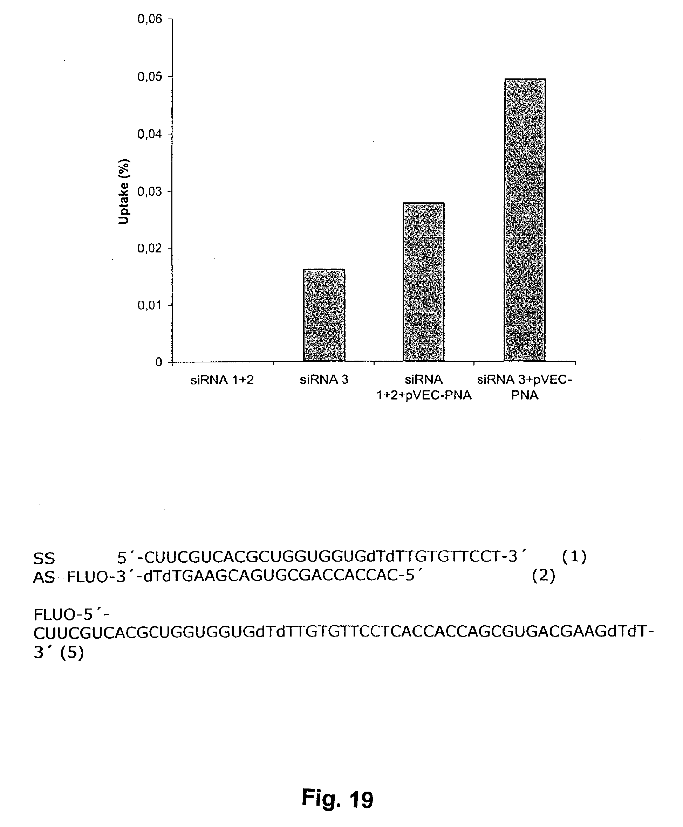

Bowes cells were grown over night in a 24 well plate (100 000 cells/well). The cells were treated with 200 µl, 1 µM fluorescently labelled peptide (pVEC), or fluorescently labelled siRNA-peptide (according to Fig 19) for 30 min at 37°C. The cells were exposed to 3 times diluted standard trypsin/EDTA to remove any peptide stuck to the outer cell membrane. The cells were then lysed with 0.1 % Triton-X and the uptake of peptide/siRNA was measured using Spectramax Gemeni XS fluorescence reader.

### Results:

As can be seen in Fig. 19, a clear difference was noted in uptake of CPP-conjugated DNA (siRNA) as compared to naked siRNA. The uptake was not due to membrane disruption by the cell-penetrating peptide. Furthermore mixture of un-conjugated pVEC and siRNA did not internalize into the cell.
As is well known in the field, siRNA has good efficiency even at low concentrations (down to pM) inside the cell, thus the uptake is considered enough to potentially activate the siRNA mediated degradation of the target mRNA.

### Example 7

### General methods in characterisation of bioactive cell-penetrating peptides

### Transwell^{™} experiments

The human colon cancer cell line Caco-2 (ATCC via LGC, Sweden) was propagated in Dulbeccos modified essential media with Glutamax (Invitrogen, Sweden) supplemented with 10 % foetal bovine serum, sodium puruvate 1 mM, non-essential amino acids 1x100, 100 U/ml penicillin and 100 µg/ml streptomycinin air enriched with 5%CO2 at 37°C.

Transwell^{™}-clear cups (0.4 µm pores, Corning Costar, The Netherlands) were coated with bovine plasma fibronectin 0.5 µg/ml (Invitrogen, Sweden). 100 000 Caco-2 cells were seeded in each cup of a 12-well Transwell^{™} (1.13 cm2 filter area) and cultured for at least ten days. The media was changed in both the lower (1.5 ml) and the upper (0.5 ml) chambers every 2-3 days. The cell confluence was examined in a phase contrast microscope and by measuring TEER with a Millicell-ERS (Millipore, Sweden) with alternating current. As controls of the cell layer permeability, FITC-labelled dextran 4,4 kDa (Sigma-Aldrich, Sweden) passage was measured with or without 10 mM EGTA treatment. Before the addition of peptides, the media in the lower well was changed to HEPES buffered Krebbs-Ringer solution (HKR). The resistance reached 600 Ω/cm2 before the experiments were initiated, values over 500 Ω/cm2 are considered as high resistance.

Fluorophore-labelled peptide at 10 µM concentration, dissolved in phosphate buffered saline (PBS) was added to the upper Transwell^{™} chamber. At each time point, a 150 µl sample was collected from the lower chamber, and the fluorescence was measured at 320/420 nm (Abz) and 492/520nm (fluorescein) on a Spectramax Gemini XS (Molecular Devices, CA).

### Cellular penetration studies and fluorescence microscopy

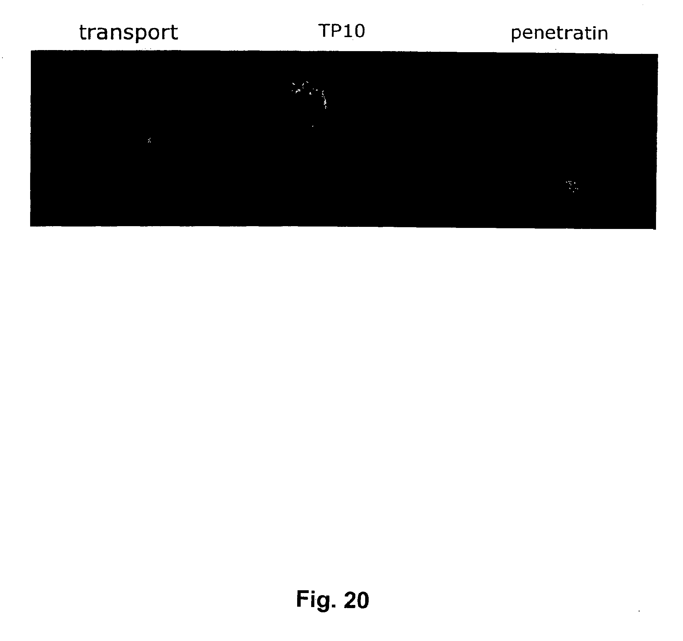

The cells were grown on round glass cover slips (12 mm, GTF, Sweden) in a 24-well plate to approximately 50 % confluence. The media was changed to serum free and the biotinylated peptide solutions were added. The cells were incubated for 30 min at 37 or 4°C. The cells were washed twice with PBS, fixed with 4% paraformaldehyde solution 15 min at room temperature (dark) and then permeabilised in 30 mM HEPES buffer containing 0.5% w/v Triton X-100, 3 min on ice. Sites for unspecific binding were blocked in PBS containing 3% (w/v) bovine serum albumin, overnight at 4°C. The peptides were visualised by staining with avidin-FITC or streptavidin-TRITC (Molecular Probes, the Netherlands). The cell nuclei were stained with Hoechst 33258 (0,5 µg/ml) for 5 min, after which the cover slips were washed 3 times with PBS and mounted in 25% glycerol in PBS. The images were obtained with a Leica DM IRE2 fluorescence microscope (Leica Microsystem., Sweden) and processed in PhotoShop 6.0 software (Adobe Systems Inc., CA) (See e.g. Fig. 20-22).

### Peptide uptake and outflow studies in cells in suspension

Cells were detached with trypsin (Invitrogen, Sweden), dissolved in culture media and centrifuged (1000 xg for 10 min at RT). The cells were resuspended, counted and aliqoted in HKR on ice, 300 000 cells /tube. Abz-labelled peptide was incubated for 15 and 30 min together with the cells in suspension, on a shaking 37 °C water bath. To stop the uptake or outflow, trypsin solution was added for 3 min. The cells were spun down at 1000 x g for 10 min at 4°C. The pellets were resuspended in HKR for fluorescence detection, or for the outflow samples, incubated again with peptide-free HKR. Fluorescence was read at 320/420 nm on a Spectramax Gemini XS (Molecular Devices, CA). The intracellular concentrations were calculated from a standard curve of Abz-labelled peptides. The average cell volume of Caco-2 cells was determined by using a Coulter 256 channelizer (Coulter Electronics Ltd. CA).

### Degradation of peptides / uptake in cells detected by mass spectrometry

Eighty percent confluent cells in 35-mm cell culture dishes were treated with 10 µM peptide dissolved in serum-free media for different time points. The cells were washed three times with PBS and cell lysates were prepared by treating the cells with 0.1 % HCl for 15 min on ice. The lysates were centrifuged at 13,000g for 5 min and frozen. Before loading, the samples were purified by using C 18 Zip Tip columns (Millipore, Sweden) and then analysed on a Voyager-DR STR system (Applied Biosystems, Framingham).

### Membrane disturbance assays

### 2-deoxyglucose assay

Cells were seeded in 12-well plates and used for experiment five days after seeding. First, 0.5 µCi of 2-deoxy-D-[1-3H]-glucose was added to each well (Amersham Pharmacia Biotech, UK) in glucose-free buffer. After 20 min incubaction at 37oC peptides were added in serum-free medium to reach the final concentrations of 5, 10 and 20µM. As a positive control, cells were treated with 1% Triton X-100 in PBS (to establish the upper boundary of leakage). At 1, 5, 15 and 30 min a 150 µl media samples were collected and Emulsifier Safe scintillation cocktail (Packard, Netherlands) was added and the radioactivity was measured in a Packard 3255 liquid scintillation counter. The relative radioactive efflux from each well was calculated as percentage of untreated cells.

### Lactate dehydrogenase assay

Lactate dehydrogenase leakage was performed using Cyclo Tox-ONE^{™} Homeogeneous Membrane Integrity Assay from Promega Corp. (Promega, Madison, WI) and the lactate dehydrogenase activity calculated according to the manufacturer's instructions.

### Histamine release assays

### Cell culturing.

RBL-2H3 cells were obtained from American Type Culture Collection (Manassas, VA). The cells were cultured in Minimal Essential Medium with Earle's salts complemented with Glutamax-I, non-essential amino acids, sodium pyruvate, penicillin, streptomycin and heat-inactivated foetal bovine serum (10%). All media components were from Invitrogen Corporation (Paisley, UK). Trypsin/EDTA was from PAA laboratories Gmbh (Linz, Austria). Cells were splitted twice a week, seeding circa 1.2 million cells per T75. For the histamine release assay, 125 000 - 250 000 cells (in 1 ml) were seeded per a well in a 24-well-plate a day before the experiment.

### Histamine release

The following solutions were used: assay buffer (10 mM HEPES, 140 mM NaCl, 5 mM KCI, 0.6 mM MgCl2, 1 mM CaCl2, 5.5 mM glucose, pH 7.4), 1 M NaOH, 10 mg/ml o-phthaldialdehyde (OPT) in methanol, 3.M HCl and 0.1% Triton X-100. All chemicals were from Sigma-Aldrich (St. Louis, MA). 1 mM peptide stock solutions were prepared in water and diluted further using assay buffer.
The cells were washed twice with assay buffer and then the peptide was added at desired concentration in 300µl of assay buffer. For the determination of the total cellular histamine, some wells were exposed to 0.1% Triton X-100. After 20 min incubation at 370C, the exposure medium was transferred to a 1.5 ml polypropylene tube and centrifuged briefly (2 min at 3000 rpm).

### Histamine determination using OPT

OPT was from Sigma-Aldrich Corp., St. Louis, MO. 200µl of the supernatant was transferred into a black untreated microwell plate (NUNC) and assayed for histamine by adding 40 µl of 1 M NaOH and 10 µl of OPT solution and shaking for 4 minutes. To terminate the reaction, 20 µl of 3 M HCl was added. After 30 seconds, the fluorescence intensity was measured using a 355 nm excitation filter and a 455 nm emission filter (SPECTRAmax®GEMINI XS, Molecular Devices, Sunnyvale, CA). The histamine released was expressed as a percentage of total cellular histamine.

### Histamine determination using ELISA

The ELISA kit for the histamine was from IBL GmbH (Hamburg, Germany). The assay was performed following the manufacturers instructions. The absorbance measurements were performed using Digiscan Microplate Reader from ASYS Hitech GmbH (Eugendorf, Austria).

### LIST OF REFERENCES

1. Lindgren, M., Hällbrink, M., Prochiantz, A. & Langel, Ü. Cell-penetrating peptides. Trends Pharmacol. Sci. 21, 99-103 (2000).
2. Derossi, D., Chassaing, G. & Prochiantz, A. Trojan peptides: the penetratin system for intracellular delivery. Trends Cell. Biol. 8, 84-87 (1998).
3. Derossi, D., Joliot, A.H., Chassaing, G. & Prochiantz, A. The third helix of the Antennapedia homeodomain translocates through biological membranes. J. Biol. Chem. 269, 10444-10450 (1994).
4. Prochiantz, A. Homeodomain-derived peptides. In and out of the cells. Ann N Y Acad Sci 886, 172-9 (1999).
5. Sandberg, M., Eriksson, L., Jonsson, J., Sjöström, M. & Wold, S. New chemical descriptors relevant for the design of biologically active peptides. A multivariate characterization of 87 amino acids. J. Med. Chem. 41, 2481-2491 (1998).
6. Altschul, S.F., Gish, W., Miller, W., Myers, E.W. & Lipman, D.J. Basic local alignment search tool. J Mol Biol 215, 403-10. (1990).
7. Ma, H. & Diamond, S.L. Nonviral gene therapy and its delivery systems. Curr Pharm Biotechnol 2, 1-17. (2001).
8. Garnett, M.C. Gene-delivery systems using cationic polymers. Crit Rev Ther Drug Carrier Syst 16, 147-207 (1999).
9. Somiari, S. et al. Theory and in vivo application of electroporative gene delivery. Mol Ther 2, 178-87. (2000).
10. Lee, R.J. & Huang, L. Lipidic vector systems for gene transfer. Crit. Rev. Therap. Drug Carrier Syst. 14, 173-206 (1997).
11. Ropert, C. Liposomes as a gene delivery system. Braz J Med Biol Res 32, 163-9. (1999).
12. Luo, D. & Saltzman, W.M. Enhancement of transfection by physical concentration of DNA at the cell surface. Nat Biotechnol 18, 893-5.
13. Felgner, P.L. et al. Nomenclature for synthetic gene delivery systems. Hum Gene Ther 8, 511-2. (1997).
14. Gebhart, C.L. & Kabanov, A.V. Evaluation of polyplexes as gene transfer agents. J Control Release 73, 401-16. (2001).
15. Boussif, O. et al. A versatile vector for gene and oligonucleotide transfer into cells in culture and in vivo: polyethylenimine. Proc Natl Acad Sci U S A 92, 7297-301. (1995).
16. Abdallah, B. et al. A powerful nonviral vector for in vivo gene transfer into the adult mammalian brain: polyethylenimine. Hum Gene Ther 7, 1947-54. (1996).
17. Schatzlein, A.G. Non-viral vectors in cancer gene therapy: principles and progress. Anticancer Drugs 12, 275-304. (2001).
18. Remy-Kristensen, A., Clamme, J.P., Vuilleumier, C., Kuhry, J.G. & Mely, Y. Role of endocytosis in the transfection of L929 fibroblasts by polyethylenimine/DNA complexes. Biochim Biophys Acta 1514, 21-32. (2001).
19. Rozanov, D.V. et al. Mutation analysis of membrane type-1 matrix metalloproteinase (MT1- MMP). The role of the cytoplasmic tail Cys(574), the active site Glu(240), and furin cleavage motifs in oligomerization, processing, and self-proteolysis of MT1-MMP expressed in breast carcinoma cells. J Biol Chem 276, 25705-14. (2001).
20. Smith, L.E., Parks, K.K., Hasegawa, L.S., Eastmond, D.A. & Grosovsky, A.J. Targeted breakage of paracentromeric heterochromatin induces chromosomal instability. Mutagenesis 13, 435-43. (1998).
21. Tang, W. et al. Development and evaluation of high throughput functional assay methods for HERG potassium channel. J Biomol Screen 6, 325-31. (2001).
22. Hallbrink, M. et al. Cargo delivery kinetics of cell-penetrating peptides. Biochim Biophys Acta 1515, 101-9. (2001).
23. Vidal, P. et al. Interactions of primary amphipathic vector peptides with membranes. Conformational consequences and influence on cellular localization. J Membr Biol 162, 259-64. (1998).
24. Kilk, K. et al. Cellular internalization of a cargo complex with a novel peptide derived from the third helix of the islet-1 homeodomain. Comparison with the penetratin peptide. Bioconjug Chem 12, 911-6. (2001).
25. Magzoub, M., Kilk, K., Eriksson, L.E., Langel, U. & Graslund, A. Interaction and structure induction of cell-penetrating peptides in the presence of phospholipid vesicles. Biochim Biophys Acta 1512, 77-89. (2001).
26. Inoue, A., Takahashi, M., Hatta, K., Hotta, Y. & Okamoto, H. Developmental regulation of islet-1 mRNA expression during neuronal differentiation in embryonic zebrafish. Dev Dyn 199, 1-11. (1994).
27. Langel, U., Land, T. & Bartfai, T. Design of chimeric peptide ligands to galanin receptors and substance P receptors. Int J Pept Protein Res 39, 516-22. (1992).
28. McKenzie, F.R. Signal Transduction (Milligan, E., Ed.), Oxford University Press, Oxford, NY, Tokyo. (1992).
29. Lowry, O.H., Rosenbrough, n.Y., Farr, A.L. & Randall, R.J. J. Biol. Chem. 193, 265-275 (1951).
30. Cassel, D. & Selinger, Z. (1976) 452(2), 538-51. 452, 538-551 (1976).
31. Sternlicht MD, Werb Z., Annu Rev Cell Dev Biol 2001;17:463-516 How matrix metalloproteinases regulate cell behavior.
32. Leppert D, Lindberg RL, Kappos L, Leib SL., Brain Res Brain Res Rev 2001 Oct;36(2-3):249-57Matrix metalloproteinases: multifunctional effectors of inflammation in multiple sclerosis and bacterial meningitis.
33. Rozanov DV, Deryugina El, Ratnikov Bl, Monosov EZ, Marchenko GN, Quigley JP, Strongin AY., J Biol Chem 2001 Jul 13;276(28):25705-14, Mutation analysis of membrane type-1 matrix metalloproteinase (MT1-MMP). The role of the cytoplasmic tail Cys(574), the active site Glu(240), and furin cleavage motifs in oligomerization, processing, and self-proteolysis of MT1-MMP expressed in breast carcinoma cells.
34. Hoekstra R, Eskens FA, Verweij J, Oncologist 2001;6(5):415-27, Matrix metalloproteinase inhibitors: current developments and future perspectives.
35. Hornebeck W, Emonard H, Monboisse JC, Bellon G., Semin Cancer Biol 2002 Jun;12(3):231-41, Matrix-directed regulation of pericellular proteolysis and tumour progression.
36. Nabeshima K, Inoue T, Shimao Y, Sameshima T, Pathol Int 2002 Apr;52(4):255-64 Matrix metalloproteinases in tumour invasion: role for cell migration.
37. Galvez BG, Matias-Roman S, Albar JP, Sanchez-Madrid F, Arroyo AG, J Biol Chem 2001 Oct 5;276(40):37491-500, Membrane type 1-matrix metalloproteinase is activated during migration of human endothelial cells and modulates endothelial motility and matrix remodeling.
38. Sounni NE, Baramova EN, Munaut C, Maquoi E, Frankenne F, Foidart JM, Noel A., Int J Cancer 2002 Mar 1;98(1):23-8, Expression of membrane type 1 matrix metalloproteinase (MT1-MMP) in A2058 melanoma cells is associated with MMP-2 activation and increased tumour growth and vascularization.
39. Chen CC, Chen N, Lau LF., J Biol Chem 2001 Mar 30;276(13):10443-52, The angiogenic factors Cyr61 and connective tissue growth factor induce adhesive signaling in primary human skin fibroblasts.
40. Laakkonen P, Porkka K, Hoffman JA, Ruoslahti E, Nat Med 2002 Jul;8(7):751-5, A tumour-homing peptide with a targeting specificity related to lymphatic vessels.
41. Pooga M., et al., 1998, FASEB J. 1998 Jan;12(1):67-77, Cell penetration by transportan.
42. Pooga M., et al., Nat Biotechnol. 1998 Sep;16(9):857-61, Cell-penetrating PNA constructs regulate galanin receptor levels and modify pain transmission in vivo.
43. Moroianu J., J.Cell Biochem, 1999, Suppl. 32-33:76-83, Nuclear import and export pathways.
44. Dayhoff, Schwartz, and Orcutt (1978) Atlas Protein Seq. Struc. 5:345-352
45. Henikoff and Henikoff (1992) Proc Natl Acad Sci U S A 89(22):10915-9.
46. Simon RJ et al., PNAS (1992) 89(20), 9367-9371 and Horwell DC, Trends Biotechnol. (1995) 13(4), 132-134.

## Claims

1. A cell-selective delivery system for a cargo, comprising a) a protease consensus site for a protease specifically overexpressed in a target cell, b) a cell-penetrating peptide and/or a non-peptide analogue thereof, and c) a cargo, wherein said cell-selective delivery system additionally comprises an inactivation sequence which represses the cellular penetration capacity of said cell-penetrating peptide, and which is cleaved by said protease.

2. The cell-selective delivery system according to claim 1, wherein the cell-penetrating peptide and/or a non-peptide analogue thereof comprises the protease consensus site.

3. The cell-selective delivery system according to claim 1 or 2, wherein the cargo is a cytostatic and/or cytotoxic agent.

4. The cell-selective delivery system according to claim 1, wherein the cell-penetrating peptide and/or a non-peptide analogue thereof is selected from the group consisting of YTAIAWVKAFIRKLRK (SEQ ID NO: 3), LRSWVISRSIRKAA (SEQ ID NO: 5), LRSWIRRLIKAWKS (SEQ lD NO: 6 ), LRSWRVIIRNGQR (SEQ lD NO: 7) and IRQIKIWFQNRRMKWKK (SEQ ID NO: 20).

5. The cell-selective delivery system according to claim 2, wherein the cell-penetrating peptide and/or a non-peptide analogue thereof comprising the protease consensus site is SGESLAY-YTAIAWVKAFIRKLRK (SEQ ID NO: 4),GPLG-LRSWVISRSIRKAA (SEQ ID NO: 8), GPLG-LRSWIRRLIKAWKS (SEQ ID NO: 9), GPLG-LRSWRVIIRNGQR (SEQ ID NO: 10), and DEEQERSEN-IRQIKIWFQNRRMKWKK (SEQ ID NO: 13).

6. The cell-selective delivery system according to any one of claims 1-5, wherein said overexpressed protease is a Zn²⁺metallo endopeptidase selected from the group consisting of MMP-1, MMP-2, and MMP-MT1.

7. The cell-selective delivery system according to any one of claims 1-5, wherein said overexpressed protease is selected from the group consisting of bacterial surface proteases and viral enzymes.

8. The cell-selective delivery system according to any one of claims 1 - 7 for use in the manufacture of a medicament.

## Patentansprüche

1. Zellselektives Zufuhrsystem für eine Beladung, umfassend a) eine Proteasekonsensusstelle für eine Protease, die in einer Zielzelle spezifisch überexprimiert wird, b) ein zelleindringendes Peptid und/oder ein Nicht-Peptidanalog davon und c) eine Beladung, wobei das zellselektive Zufuhrsystem zusätzlich eine Inaktivierungssequenz umfaßt, die die zelluläre Penetrationsfähigkeit des zelleindringenden Peptids unterdrückt und die durch die Protease gespalten wird.

2. Zellselektives Zufuhrsystem gemäß Anspruch 1, wobei das zelleindringende Peptid und/oder ein Nicht-Peptidanalog davon die Proteasekonsensusstelle umfaßt.

3. Zellselektives Zufuhrsystem gemäß Anspruch 1 oder 2, wobei die Beladung ein zytostatisches und/oder zytotoxisches Mittel ist.

4. Zellselektives Zufuhrsystem gemäß Anspruch 1, wobei das zelleindringende Peptid und/oder ein Nicht-Peptidanalog davon ausgewählt ist aus der Gruppe bestehend aus YTAIAWVKAFIRKLRK (SEQ ID NO: 3), LRSWVISRSIRKAA (SEQ ID NO: 5), LRSWIRRLIKAWKS (SEQ ID NO: 6), LRSWRVIIRNGQR (SEQ ID NO: 7) und IRQIKIWFQNRRMKWKK (SEQ ID NO: 20).

5. Zellselektives Zufuhrsystem gemäß Anspruch 2, wobei das zelleindringende Peptid und/oder ein Nicht-Peptidanalog davon, umfassend die Proteasekonsensusstelle SGESLAY-YTAIAWVKAFIRKLRK (SEQ ID NO: 4), GPLG-LRSWVISRSIRKAA (SEQ ID NO: 8), GPLG-LRSWIRRLIKAWKS (SEQ ID NO: 9), GPLG-LRSWRVIIRNGQR (SEQ ID NO: 10) und DEEQERSEN-IRQIKIWFQNRRMKWKK (SEQ ID NO: 13) ist.

6. Zellselektives Zufuhrsystem gemäß einem der Ansprüche 1 bis 5, wobei die überexprimierte Protease eine Zn²⁺-Metalloendopeptidase ist, ausgewählt aus der Gruppe bestehend aus MMP-1, MMP-2 und MMP-MT1.

7. Zellselektives Zufuhrsystem gemäß einem der Ansprüche 1 bis 5, wobei die überexprimierte Protease ausgewählt ist aus der Gruppe bestehend aus bakteriellen Oberflächenproteasen und viralen Enzymen.

8. Zellselektives Zufuhrsystem gemäß einem der Ansprüche 1 bis 7 zur Verwendung bei der Herstellung eines Medikaments.

## Revendications

1. Un système de délivrance d'une charge vers une cellule spécifique, comprenant a) un site consensus de protéase pour une protéase spécifiquement surexprimée dans une cellule cible, b) un peptide pénétrant dans les cellules et/ou un analogue non peptidique de celui-ci, et c) une charge ; dans lequel ledit système de délivrance vers une cellule spécifique comprend en outre une séquence d'inactivation qui réprime la capacité de pénétration cellulaire dudit peptide pénétrant dans les cellules, et qui est clivée par ladite protéase.

2. Le système de délivrance vers une cellule spécifique selon la revendication 1, dans lequel le peptide pénétrant dans les cellules et/ou un analogue non peptidique de celui-ci comprend le site consensus de protéase.

3. Le système de délivrance vers une cellule spécifique selon la revendication 1 ou 2, dans lequel la charge est un agent cytostatique ou cytotoxique.

4. Le système de délivrance vers une cellule spécifique selon la revendication 1, dans lequel le peptide pénétrant dans les cellules et/ou un analogue non peptidique de celui-ci est sélectionné dans le groupe constitué de YTAIAWVKAFIRKLRH (SEQ ID NO :3), LRSWVISRSIRKAA (SEQ ID NO: 5), LRSWIRRLIKAWKS (SEQ ID NO: 6), LRSWRVIIRNGQR (SEQ ID NO: 7) et IRQIKIWFQNRRMKWKK (SEQ ID NO :20).

5. Le système de délivrance vers une cellule spécifique selon la revendication 2, dans lequel le peptide pénétrant dans les cellules et/ou un analogue non peptidique de celui-ci comprenant le site consensus de protéase est SGESLAY-YTAIAWVKAFIRKLRH (SEQ ID NO : 4), GPLG-LRSWVISRSIRKAA (SEQ ID NO : 8), GPLG-LRSWIRRLIKAWKS (SEQ ID NO : 9), GPLG-LRSWRVIIRNGQR (SEQ ID NO : 10), et DEEQERSEN- IRQIKIWFQNRRMKWKK (SEQ ID NO : 13).

6. Le système de délivrance vers une cellule spécifique selon l'une quelconque des revendications 1 à 5, dans lequel ladite protéase surexprimée est une métallo-endopeptidase à Zn²⁺ sélectionnée dans le groupe constitué de MMP-1, MMP-2 et MMP-MT1.

7. Le système de délivrance vers une cellule spécifique selon l'une quelconque des revendications 1 à 5, dans lequel ladite protéase surexprimée est sélectionnée dans le groupe constitué des protéases de surface bactériennes et des enzymes virales.

8. Le système de délivrance vers une cellule spécifique selon l'une quelconque des revendications 1 à 7 pour une utilisation dans la fabrication d'un médicament.


### SEQUENCE LISTING

<110> CePeP AB
<120> Cell-selective delivery system
<130> 110135201
<140> EP 03760107.7 / WO20031B03163
   <141> 2003-06-18
<150> SE20020001863
   <151> 2002-06-18
<150> US20020391788P
   <151> 2002-06-25
<160> 20
<170> Patentln version 3.3
<210> 1
   <211> 16
   <212> PRT
   <213> Rattus norvegicus
<220>
   <221> PEPTIDE
   <222> (1)..(16)
   <223> Penetratin
<400> 1
   Arg Gln Ile Lys Ile Trp Phe Gln Asn Arg Arg Met Lys Trp Lys Lys
   1               5                   10                  15
<210> 2
   <211> 21
   <212> PRT
   <213> Artificial sequence
<220>
   <223> Peptide - TP10
<400> 2
   Ala Gly Tyr Leu Leu Gly Lys Ile Asn Leu Lys Ala Leu Ala Ala Leu Ala Lys Lys Ile Leu
   1               5                   10                  15                  20
<210> 3
   <211> 16
   <212> PRT
   <213> Artificial sequence
<220>
   <223> Peptide - YTA-2
<400> 3
   Tyr Thr Ala Ile Ala Trp Val Lys Ala Phe Ile Arg Lys Leu Arg Lys
   1               5                   10                  15
<210> 4
   <211> 23
   <212> PRT
   <213> Artificial sequence
<220>
   <223> Peptide YTA-2ps
<400> 4
   Ser Gly Glu Ser Leu Ala Tyr Tyr Thr Ala Ile Ala Trp Val Lys Ala Phe Ile Arg Lys Leu Arg Lys
   1 5 10 15 20
<210> 5
   <211> 14
   <212> PRT
   <213> Artificial sequence
<220>
   <223> Peptide - LRSW-1
<400> 5
   Leu Arg Ser Trp Val Ile Ser Arg Ser Ile Arg Lys Ala Ala
   1 5 10
<210> 6
   <211> 14
   <212> PRT
   <213> Artificial sequence
<220>
   <223> Peptide LRSW-2
<400> 6
   Leu Arg Ser Trp Ile Arg Arg Leu Ile Lys Ala Trp Lys Ser
   1 5 10
<210> 7
   <211> 13
   <212> PRT
   <213> Artificial sequence
<220>
   <223> Peptide-LRSW-3
<400> 7
   Leu Arg Ser Trp Arg Val Ile Ile Arg Asn Gly Gln Arg
   1 5 10
<210> 8
   <211> 18
   <212> PRT
   <213> Artificial sequence
<220>
   <223> Peptide - LRSW-1 ps
<400> 8
   Gly Pro Leu Gly Leu Arg Ser Trp Val Ile Ser Arg Ser Ile Arg Lys Ala Ala
   1 5 10 15
<210> 9
   <211> 18
   <212> PRT
   <213> Artificial sequence
<220>
   <223> Peptide LRSW-2ps
<400> 9
   Gly Pro Leu Gly Leu Arg Ser Trp Ile Arg Arg Leu Ile Lys Ala Trp Lys Ser
   1 5 10 15
<210> 10
   <211> 17
   <212> PRT
   <213> Artificial sequence
<220>
   <223> Peptide -LRSW-3ps
<400> 10
   Gly Pro Leu Gly Leu Arg Ser Trp Arg Val Ile Ile Arg Asn Gly Gln Arg
   1 5 10 15
<210> 11
   <211> 10
   <212> PRT
   <213> Artificial sequence
<220>
   <223> amino acid sequence - MMT site
<400> 11
   Ser Gly Glu Ser Leu Ala Tyr Tyr Thr Ala
   1 5 10
<210> 12
   <211> 8
   <212> PRT
   <213> Artificial sequence
<220>
   <223> amino acid sequence - MMP site
<400> 12
   Gly Pro Leu Gly Leu Arg Ser Trp
   1 5
<210> 13
   <211> 26
   <212> PRT
   <213> Artificial sequence
<220>
   <223> Peptide - SelCPP2
<400> 13
   Asp Glu Glu Gln Glu Arg Ser Glu Asn Ile Arg Gln Ile Lys Ile Trp Phe Gln Asn Arg Arg Met
   1 5 10 15 20
   Lys Trp Lys Lys
   25
<210> 14
   <211> 16
   <212> PRT
   <213> Artificial sequence
<220>
   <223> Peptide - Evo165
<400> 14
   Leu Leu Lys Lys Arg Lys Val Val Arg Leu Ile Lys Phe Leu Leu Lys
   1 5 10 15
<210> 15
   <211> 16
   <212> PRT
   <213> Artificial sequence
<220>
   <223> Peptide - Evo165b
<400> 15
   Leu Leu Lys Lys Arg Lys Val Val Arg Leu Ile Lys Gln Lys Gln Lys
   1 5 10 15
<210> 16
   <211> 15
   <212> PRT
   <213> Artificial sequence
<220>
   <223> Peptide - Evo165c
<400> 16
   Leu Leu Lys Lys Arg Lys Val Arg Leu Ile Lys Gln Lys Gln Lys
   1 5 10 15
<210> 17
   <211> 16
   <212> PRT
   <213> Artificial sequence
<220>
   <223> Peptide - Evo165d
<400> 17
   Leu Leu Lys Lys Arg Lys Val Val Arg Leu Ile Lys Ala His Ser Lys
   1 5 10 15
<210> 18
   <211> 15
   <212> PRT
   <213> Artificial sequence
<220>
   <223> Peptide - Evo165e
<400> 18
   Leu Leu Lys Lys Arg Lys Val Arg Leu Ile Lys Ala His Ser Lys
   1 5 10 15
<210> 19
   <211> 15
   <212> PRT
   <213> Artificial sequence
<220>
   <223> Peptide-Evo165f
<400> 19
   Leu Leu Lys Lys Arg Lys Val Val Arg Leu Ile Lys Val Arg Lys
   1 5 10 15
<210> 20
   <211> 17
   <212> PRT
   <213> Artificial sequence
<220>
   <223> Peptide - lle + penetratin
<400> 20
   Ile Arg Gln Ile Lys Ile Trp Phe Gln Asn Arg Arg Met Lys Trp Lys Lys
   1 5 10 15